# FASCICULE DE BREVET EUROPEEN

(11) **EP 0 585 155 B1**
(45) Date de publication et mention de la délivrance du brevet: **11.12.1996**
(21) Numéro de dépôt: 93401937.3
(22) Date de dépôt: 27.07.1993
(51) Int. Cl.: C07K 5/06, A61K 38/05

(54) **Peptides dérivés de trifluorométhylcétones ayant une actovoté inhibants d'élastase leucocytaire humaine (HLE), leur procédé de préparation et les compositions pharmaceutiques qui les contiennent**
Trifluormethylketon Peptid Derivate mit HLE-inhibierende Aktivität, deren Herstellung und pharmazeutischen Zusammensetzungen
Trifluoromethylketone derivatives of peptides having HLE inhibiting activity, their preparation and pharmaceutical composition

(30) Priorité: 28.07.1992 FR 9209254
(43) Date de publication de la demande: 02.03.1994
(73) Titulaire: ADIR ET COMPAGNIE, F-92415 Courbevoie Cédex (FR)
(72) Inventeur: Vincent, Michel, F-92220 Bagneux (FR); de Nanteuil, Guillaume, F-92150 Suresnes (FR); Remond, Georges, F-78000 Versailles (FR); Portevin, Bernard, F-78990 Elancourt (FR); Herve, Yolande, F-92800 Puteaux (FR); Lonchampt, Michel, F-94150 Rungis (FR)

(56) Documents cités:
- EP-A- 0 276 101
- EP-A- 0 369 391
- EP-A- 0 494 071
- JOURNAL OF MEDICINAL CHEMISTRY. vol. 35, no. 4 , 21 Février 1992 , WASHINGTON US pages 641 - 662 J.W.SKILES 'INHIBITION OF hle BY N-SUBSTITUTED PEPTIDYL TRIFLUOROMETHYL KETONES'

## Description

La présente invention concerne de nouveaux peptides dérivés de trifluorométhylcétones, leur procédé de préparation et les compositions pharmaceutiques qui les contiennent.

Ces nouveaux dérivés peptidiques possèdent des propriétés inhibitrices de l'élastase leucocytaire humaine.

L'élastine est une protéine fibreuse élastique du tissu conjonctif des vertébrés. Elle est présente dans les parois vasculaires, la peau, les poumons, les cartilages, les ligaments et d'autres tissus. Les élastases sont des enzymes capables de solubiliser l'élastine fibreuse. L'élastase leucocytaire humaine est une sérine protéase, qui se trouve sous forme active dans les granules azurophiles du neutrophile polymorphonucléaire. C'est une glycoprotéine de 25 à 30 kDa formée de 218 acides aminés. L'élastase leucocytaire humaine (ELH) solubilise l'élastine fibreuse, mais clive également d'autres protéines de la matrice extracellulaire (collagènes, fibronectine, protéoglycanes, ...), hydrolyse et inactive un certain nombre de protéines plasmatiques (facteur de la coagulation, immunoglobuline, complément, ...). L'activité élastolytique est contrôlée et régulée par des inhibiteurs naturels (alpha 1 - antitrypsine, alpha 2 - macroglobuline, l'inhibiteur bronchique).

Des inhibiteurs de l'élastase leucocytaire humaine, réversibles ou irréversibles ont été décrits dans la littérature pour le traitement de situations physiopathologiques où son rôle a été évoqué (D.A. TRAINOR, TIPS, 8, 303-307, 1987).

Ces états pathologiques peuvent être l'emphysème pulmonaire, l'arthrite rhumatoïde, les maladies dégénératives du tissu conjonctif comme l'athérosclérose (J.G. BIETH, "Elastases : Catalytic and Biological Properties" dans "Regulation of matrix accumulation" - R.P. MECHAM - Academic Press, NY, 217-320, 1986), le syndrome de détresse respiratoire aigue de l'adulte (P.M. SUTER et coll., Am. Rev. Respir. Dis., 145, 1016-1022, 1992), la fibrose kystique (K.C. MEYER et coll., Am. Rev. Respir. Dis., 144, 580-585, 1991), la bronchite chronique (J.A. NADEL, Respiration, 58 (suppl.1, 3-5), 1991), les glomérulonéphrites (E. SANDERS et coll., Renal. physiol., 3, 355-359, 1980), le psoriasis (J. SCHALKWIJK et coll., Br. J. Dermatology, 122, 631-644, 1990), les lésions tissulaires survenant au cours des processus d'ischémie-reperfusion (F.A. NICOLINI et coll., Am. HEART J., 122-1245, 1991 et C.R.B. WELBOURN et coll., Am. J. Physiol. 260, 1852-1856, 1991). Elle pourrait également jouer un rôle dans les phénomènes de migration cellulaire normale ou pathologique-invasion tumorale (J.G. BIETH cité plus haut).

Récemment des peptides dérivés de trifluorométhylcétones ont été décrits comme inhibiteurs d'ELH. C'est le cas plus particulièrement des composés décrits dans les brevets EP 189 305, EP 369 391, EP 276 101 et EP 494 071 ainsi que dans J. Med. Chem., 1992, 35, 641-662. La présente invention concerne plus spécifiquement les composés de formule (I) : 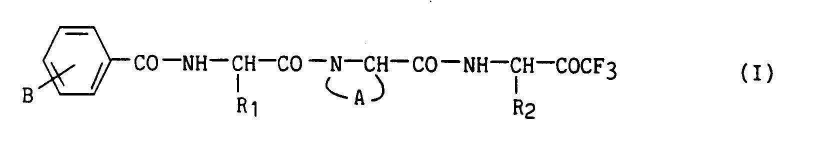 dans laquelle :
- R₁: représente un radical alkyle (C₁-C₆) linéaire ou ramifié substitué ou non par un radical cycloalkyle (C₃-C₇), phényle, amino ou benzyloxycarbonylamino, (3,5-ditertbutyl-4-hydroxy)benzylcarbonylamino, (3,5-ditertbutyl-4-hydroxy)phénylthio ou 3-[(3,5-ditertbutyl-4-hydroxy)phénylthio]propylcarbonylamino,
- R₂: représente un radical alkyle (C₁-C₆) linéaire ou ramifié,
- A: représente avec les atomes d'azote et de carbone auquel il est attaché un cycle 2-azabicyclo[2.2.2]octane, 2-azabicyclo[2.2.1] heptane, perhydroindole,
- B: représente l'un quelconque des radicaux suivants :
- a: 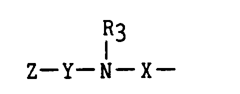 dans lequel :
- R₃: représente un atome d'hydrogène ou un radical alkyle (C₁-C₆) linéaire ou ramifié substitué ou non par un radical phényle,
- X, Y,: différents, représentent CO ou SO₂,
- Z: représente
- un radical alkyle (C₁-C₆) linéaire ou ramifié substitué par un ou deux radicaux, identiques ou différents, cycloalkyle (C₃-C₇) ou trihalogénoalkyle (C₁-C₄),
- un radical adamant-1-yl,
- un radical phényle substitué ou non par un ou plusieurs, identiques ou différents, atomes d'halogène ou radicaux alkyle (C₁-C₆) linéaire ou ramifié, hydroxy, alkoxy (C₁-C₆) linéaire ou ramifié, trihalogénoalkyle (C₁-C₄), cyano, 1,4-dihydropyridin-4-yl (substitué ou non par un ou plusieurs radicaux, indentiques ou différents, alkyle (C₁-C₆) linéaire ou ramifié ou alkoxycarbonyle (C₁-C₆) linéaire ou ramifié), (3,5-ditertbutyl-4-hydroxy) benzylcarbonylaminosulfonyl, (3,5-ditertbutyl-4-hydroxy) benzoyloxy, (3-éthoxy-2-hydroxy)propoxy, ou [3,5-ditertbutyl-4-(éthoxyméthoxy)]benzyloxy,
- ou, un radical alkényle (C₁-C₄) substitué par un radical [3,5-ditertbutyl-4-(éthoxyméthoxy)]phényle ou (3,5-ditertbutyl-4-hydroxy)phényle,
- c: 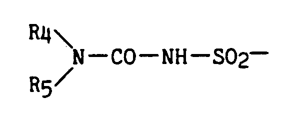 dans lequel :
- R₅: représente un atome d'hydrogène,
- R₄,: représente phényle (substitué ou non par un ou plusieurs atomes d'halogène, radical alkyle (C₁-C₆) linéaire ou ramifié, alkoxy (C₁-C₆) linéaire ou ramifié, trihalogénoalkyle (C₁-C₄)),
- ou: forment avec l'atome d'azote auxquels ils sont attachés le cycle :
- 3-azabicyclo[3.3.0]octane,
- f₁: 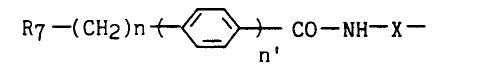
- f₂: R₇―(CH₂)ₙ―
dans lesquels :
- X: est tel que défini précédemment,
- n: est égal à 0, 1, 2 ou 3,
- n': est égal à 0 ou 1,
- R₇: représente un radical :
. 3,5-ditertbutyl-4-hydroxy phényle,
. 3,5-ditertbutyl-4-hydroxy phénoxy,
. ou 3,5-ditertbutyl-4-hydroxy phénylthio,
- h: 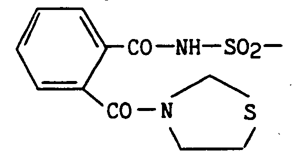
composés de formule (I) qui comprennent les hydrates de la fonction cétone COCF₃ correspondants,
leurs énantiomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, sulfurique, tartrique, maléique, fumarique, méthane sulfonique, camphorique, etc...

Parmi les bases pharmaceutiquement acceptables, on peut citer à titre non limitatif l'hydroxyde de sodium, l'hydroxyde de potassium, la tertbutylamine, la diéthylamine, l'éthylènediamine, etc...

L'invention s'étend également au procédé de préparation des composés de formule (I) caractérisé en ce que l'on utilise, comme produit de départ un alcool de formule (II), dont on a éventuellement séparé les isomères par une technique classique de séparation : 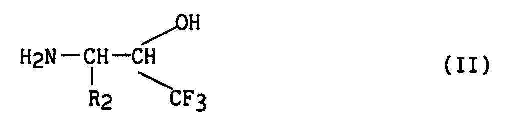 dans laquelle R₂ a la même signification que dans la formule (I), que l'on fait réagir :
- a: soit sur un amino-acide protégé de formule (III), dont on a éventuellement séparé les isomères selon une technique classique de séparation, par une technique classique de couplage peptidique comme celle décrite par W. KONIG et R. GEIGER (Ber., 103, 788, 1970) : 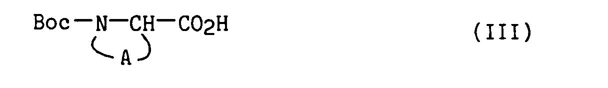 dans laquelle A a la même signification que dans la formule (I) et Boc représente un groupement tertbutoxycarbonyle, pour conduire au composé de formule (IV) : 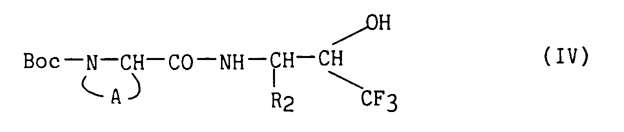 dans laquelle A, R₂ et Boc ont la même signification que précédemment,
composé de formule (IV) :
* ou bien que l'on déprotège par hydrolyse acide pour conduire au composé de formule (V) : 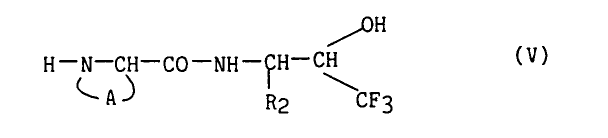 dans laquelle A et R₂ ont la même signification que précédemment,
   sur lequel on fait réagir un amino-acide protégé de formule (VI), dont on a éventuellement séparé les isomères selon une technique classique de séparation,
   en présence d'un agent de couplage classique de la synthèse peptidique : 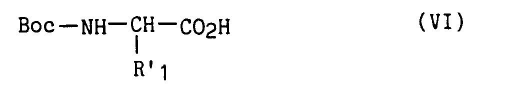 dans laquelle Boc représente un radical butoxycarbonyle et R'₁ représente un radical alkyle (C₁-C₆) linéaire ou ramifié substitué ou non par un radical cycloalkyle (C₃-C₇), phényle ou benzyloxycarbonylamino,
   pour conduire au composé de formule (VII) : 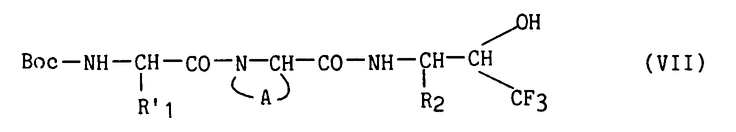 dans laquelle Boc, R'₁, A et R₂ ont la même signification que précédemment,
   qui subit une oxydation pour conduire au composé de formule (VIII), dont on sépare éventuellement les isomères selon une technique classique de séparation, 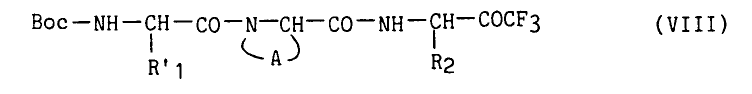 dans laquelle Boc, R'₁, A et R₂ ont la même signification que précédemment,
* ou bien qui subit une oxydation pour conduire au composé de formule (X) : 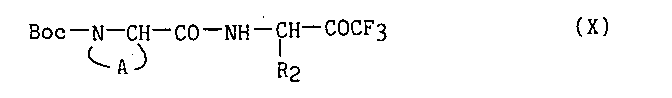 dans laquelle Boc, A et R₂ ont la même signification que précédemment,
   que l'on déprotège en milieu acide pour conduire au composé de formule (XI) : 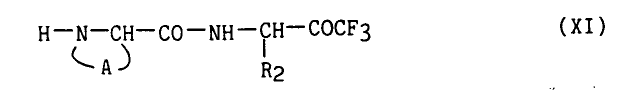 dans laquelle A et R₂ ont la même signification que précédemment,
   que l'on fait réagir sur un amino-acide protégé de formule (VI) tel que défini précédemment,
   pour conduire au composé de formule (VIII) défini précédemmment,
- b: soit sur un dipeptide protégé de formule (XII), obtenu par couplage classique de deux amino-acides sous forme racémique ou d'énantiomères purs, 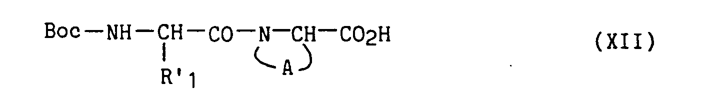 dans laquelle Boc, R'₁ et A ont la même signification que précédemment,
pour conduire au composé de formule (VII) défini précédemment,
qui subit une oxydation et conduit au composé de formule (VIII) défini précédemment,
composé de formule (VIII) que l'on déprotège en milieu acide,
pour conduire au composé de formule (IX), dont on sépare éventuellement les isomères selon une technique classique de séparation, 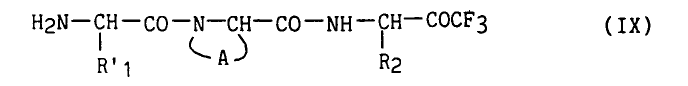
dans laquelle R'₁, A et R₂ ont la même signification que précédemment,
que l'on fait réagir sur un acide de formule (XIII), selon une technique classique de couplage peptidique, 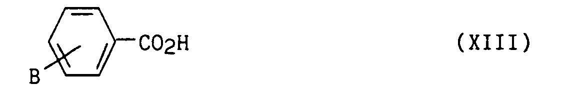
dans laquelle B a la même signification que dans la formule (I),
pour conduire au composé de formule (I/a), cas particulier des composés de formule (I), 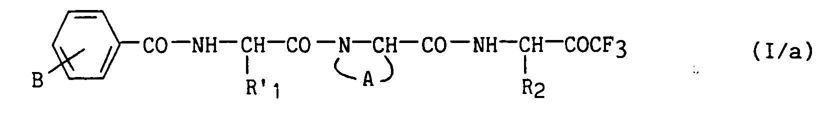
dans laquelle B, A, R'₁ et R₂ ont la même signification que précédemment,
qui, lorsque R'₁ représente un radical alkyle substitué par un groupement benzyloxycarbonylamino, est déprotégé, si on le souhaite, par hydrogénation catalytique,
pour conduire au composé de formule (I/b), cas particulier des composés de formule (I), 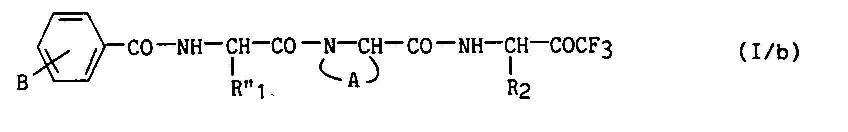
dans laquelle B, A et R₂ ont la même signification que dans la formule (I) et R''₁ représente un alkyle substitué par un radical amino,
composés de formule (I/a) et (I/b), que l'on purifie selon une technique classique de purification, dont on sépare, si on le désire, les isomères selon une technique classique de séparation, puis, si nécessaire, que l'on transforme en sel d'addition à un acide ou à une base pharmaceutiquement acceptable.

Les composés de formule (I) possèdent des propriétés pharmacologiques très intéressantes, en particulier inhibitrices de l'élastase leucocytaire humaine. A ce titre, ils peuvent être utilisés avec profit dans un certain nombre d'indications thérapeutiques comme l'emphysème pulmonaire, la bronchite chronique, le syndrome de détresse respiratoire aigue de l'adulte, la fibrose kystique, l'arthrite rhumatoïde, la glomérulonéphrite, les inflammations, les syndromes d'ischémie reperfusion, les phénomènes d'invasion et de diffusion des cellules malignes, les maladies dégénératives du tissu conjonctif, le vieillissement cutané.

L'activité inhibitrice de l'élastase leucocytaire humaine a été démontrée sur des tests in vitro et in vivo. Les composés ont présentés des activités inhibitrices supérieures aux produits de références tel que la chloromethylcétone ou la dichloroisocoumarine.

Les substituants des composés de formule (I) ont permis d'ajouter à l'activité inhibitrice d'élastase leucocytaire humaine, des propriétés de type anti-inflammatoire, anti-radicalaire, et/ou mucorégulatrice.

La présente invention a également pour objet les compositions pharmaceutiques renfermant comme principe actif au moins un composé de formule générale (I) ou un de ses sels d'addition à un acide ou une base pharmacologiquement acceptable seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, les aérosols, les ampoules buvables et injectables...

La posologie utile varie selon l'âge et le poids du patient, la nature et la sévérité de l'affection ainsi que la voie d'administration.

Celle-ci peut être orale, nasale, rectale ou parentérale. D'une manière générale, la posologie unitaire s'échelonne entre 10 µg et 300 mg pour un traitement en 1 ou 3 prises par 24 heures.

Une voie préférentielle d'administration des dérivés de l'invention est la voie aérosol sous forme de poudre ou d'aérosol liquide.

Les exemples suivants illustrent l'invention et ne la limitent en aucune façon.

Les produits de départ utilisés sont des produits connus ou préparés selon des modes opératoires connus.
Les abréviations utilisées dans les exemples sont les suivantes :
- Abo: à la place de 2-azabicyclo[2.2.2]octane-3-carbonyl
- Boc: à la place de tertbutoxycarbonyl
- Val: à la place de valyle
- Phi: à la place de perhydroindole-2-carbonyl
- Abh: à la place de 2-azabicyclo[2.2.1]heptane-3-carbonyl
- Lys: à la place de lysyle
- Cys: à la place de cystéinyle

Les préparations A à V décrivent des produits de départ utiles dans la synthèse des composés de formule (I).

### Préparation A : Acide 4-(4-chlorobenzoylaminosulfonyl)benzoïque

A une solution contenant 60 mmoles d'acide parachlorobenzoïque dans 100 ml de diméthylformamide (DMF) sont ajoutées successivement 60 mmoles de 4-carbéthoxybenzènesulfonamide, une solution contenant 60 mmoles de 4-diméthylaminopyridine dans 40 ml de DMF. L'ensemble est maintenu 20 heures sous agitation à température ambiante, filtré puis évaporé. L'huile obtenue est purifiée par chromatographie sur colonne de silice en utilisant comme solvant un mélange dichlorométhane/éthanol (93/7) et conduit au 4-(4-chlorobenzoylaminosulfonyl)benzoate d'éthyle qui est saponifié par agitation pendant 20 heures dans un mélange contenant 48 ml de soude 1N et 60 ml d'éthanol. Après évaporation, addition de 100 ml d'eau puis de 50 ml d'acide chlorhydrique 1N, le produit attendu précipite et est filtré, lavé à l'eau et au dichlorométhane.
Point de fusion : 265°C

### Préparation B : Acide 4-[(2-oxotétrahydrothiophèn-3-yl)aminosulfonyl]benzoïque

A 50 mmoles d'acide 4-chlorosulfonylbenzoïque dans 120 ml de dioxane, on ajoute simultanément à température ambiante 50 mmoles de chlorhydrate d'homocystéinethiolactone et 100 mmoles de triéthylamine dans 30 ml de dioxane. L'agitation est maintenue 18 heures et le solvant évaporé. Le résidu est repris par de l'eau et extrait par de l'acétate d'éthyle. Après séchage et évaporation, le produit attendu est obtenu après purification sur colonne de silice en utilisant comme éluant un mélange dichlorométhane/acétate d'éthyle (90/10).
Point de fusion : 146°C

### Préparation C : Acide 4-[2-(1-oxa-2-oxo-3,8-diazaspiro[4.5]déc-8-yl)éthyl]benzoïque

100 mmoles d'acide 4-(2-chloroéthyl)benzoïque et 100 mmoles de 1-oxa-2-oxo-3,8-diazaspiro[4.5]décane sont mises en solution dans 300 ml de méthylisobutylcétone en présence de 300 mmoles de carbonate de potassium et de 0,25 g d'iodure de potassium. L'ensemble est porté 30 heures à reflux, sous agitation, puis évaporé. Le résidu est repris à l'eau et lavé par de l'acétate d'éthyle. La phase aqueuse est acidifiée par de l'acide chlorhydrique concentré (pH = 1), filtrée et le produit attendu est fixé sur résine, lavé à l'eau et élué par de l'ammoniaque à 20 %. Après évaporation, le produit attendu est obtenu après purification sur colonne de silice en utilisant comme éluant un mélange acétone/eau (90/10).
Point de fusion : > 250°C

### Préparation D : Acide 4-[2-(4-hydroxy-3,5-ditertbutylphénylthio)éthyl]benzoïque

A une solution contenant 50 mmoles d'éthylate de sodium dans 200 ml d'éthanol, sont ajoutées, sous agitation et sous atmosphère inerte, 50 mmoles de 4-hydroxy-3,5-ditertbutylthiophénol, 50 mmoles d'acide 4-(2-chloroéthyl)benzoïque et 100 ml de diméthylformamide. L'ensemble est chauffé à 65-70°C pendant 8 heures. L'éthanol est évaporé, la phase restante reprise par de l'acétate d'éthyle, filtrée et évaporée. Le produit attendu est obtenu par purification du résidu par chromatographie sur gel de silice en utilisant comme éluant un mélange dichlorométhane/dioxane (95/5).
Point de fusion : 147°C

| Microanalyse élémentaire : | | | |
|---|---|---|---|
| | C % | H % | S % |
| calculé | 71,47 | 7,82 | 8,29 |
| trouvé | 71,01 | 8,04 | 8,02 |

### Préparation E : Acide 4-[4-[2-(4-hydroxy-3,5-ditertbutylphénylthio)éthyl]benzoylaminosulfonyl]benzoïque

A 6 mmoles du composé obtenu dans la préparation D dans 70 ml de tétrahydrofurane (THF) anhydre, sous atmosphère inerte, on ajoute 6 mmoles de N-méthylmorpholine et l'ensemble est refroidi à 5°C. 6 mmoles de chloroformiate d'isobutyle en solution dans 10 ml de THF anhydre sont alors ajoutées lentement. Après retour à température ambiante, l'ensemble est agité 30 minutes. 6 mmoles de 4-carbéthoxyphénylsulfonamide dans 25 ml de THF anhydre sont alors additionnées lentement et l'agitation maintenue 18 heures. Après évaporation, le résidu est repris par de l'acétate d'éthyle, lavé par une solution saturée de bicarbonate de sodium, par de l'acide citrique à 10 % et par de l'eau, séché et évaporé. L'ester ainsi obtenu est purifié par chromatographie sur colonne de silice en utilisant comme solvant d'élution un mélange dichlorométhane/méthanol/ammoniaque (90/10/1). Le produit attendu est alors obtenu par saponification de l'ester.

### Préparation F : Acide 4-[(2,5,7,8-tétraméthyl-6-hydroxychroman-2-yl)carbonylaminosulfonyl]benzoïque

Le produit attendu est obtenu en utilisant le même procédé que celui décrit dans la préparation A.
Point de fusion : 128°C

### Préparation G : Acide 4-[S-Tertbutyl-N-acétyl cystéinylaminosulfonyl)benzoïque

Le produit attendu est obtenu en utilisant le même procédé que celui décrit dans la préparation A.
Point de fusion : 227°C

### Préparation H : Acide 4-[2-[(1,3-thiazolidin-3-yl)carbonyl]benzoylaminosulfonyl]benzoïque

Le produit attendu est obtenu en utilisant le même procédé que celui décrit dans la préparation A.
Point de fusion : > 250°C

### Préparation I : Acide 4-[(4-hydroxy-2-méthyl-1,1-dioxo-1,2-benzothiazin-3-yl)carbonyl]benzoïque

### Stade A : N-[(4-carbéthoxybenzoyl)méthyl]saccharine

45 mmoles de 4-bromoacétylbenzoate d'éthyle dans 120 ml de diméthylformamide et 48 mmoles de saccharinate de sodium sont portées à 100°C pendant 180 minutes. Après évaporation du solvant, reprise par 200 ml d'eau, extraction au dichlorométhane, séchage et évaporation, le résidu est repris par de l'oxyde d'isopropyle puis filtré et conduit au produit attendu.

### Stade B : 3-(4-Carbéthoxybenzoyl)-1,1-dioxo-1,2-benzothiazin-4-one

Le produit attendu est obtenu à partir du composé décrit au stade A selon la méthode décrite par H. ZINNER et Coll. (J.O.C., 30, 2241-2246, 1965).
Point de fusion : 161°C

### Stade C : 3-(4-Carbéthoxybenzoyl)-2-méthyl-1,1-dioxo-1,2-benzothiazin-4-one

Le produit attendu est obtenu à partir du composé décrit au stade B selon la méthode décrite par H. ZINNER et Coll. (J.O.C., 30, 2241-2246, 1965).

### Stade D : Acide 4-[(4-hydroxy-2-méthyl-1,1-dioxo-1,2-benzothiazin-3-yl)carbonyl]benzoïque

Le produit attendu est obtenu par saponification de 7 mmoles du composé obtenu au stade précédent dans 30 ml de soude 0,5 N et 15 ml d'éthanol, pendant 48 heures. Après évaporation de l'éthanol, acidification par 20 ml d'acide chlorhydrique 1N, le précipité est filtré et lavé à l'eau.
Point de fusion : > 260°C

### Préparation J : Acide 4-[(dicyclopropylméthyl)carbonylaminosulfonyl]benzoïque

Le produit attendu est obtenu en utilisant le même procédé que celui décrit dans la préparation A en utilisant comme agent de couplage la dicyclohexylcarbodiimide.

| Microanalyse élémentaire : | | | | |
|---|---|---|---|---|
| | C % | H % | N % | S % |
| calculé | 55,72 | 5,30 | 4,33 | 9,92 |
| trouvé | 55,93 | 5,38 | 4,89 | 9,99 |

### Préparation K : Acide 4-[(4-chlorophényl)uréidosulfonyl]benzoïque

Le composé a été obtenu selon la technique décrite dans Synthesis, 3, 221, 1990.

| Microanalyse élémentaire : | | | | |
|---|---|---|---|---|
| | C % | H % | N % | S % |
| calculé | 50,20 | 3,95 | 7,32 | 8,38 |
| trouvé | 50,30 | 3,96 | 7,53 | 8,15 |

### Préparation L : Acide 4-[(adamant-1-yl)carbonylaminosulfonyl]benzoïque

25 mmoles du chlorure de l'acide 1-adamantane carboxylique et 25 mmoles de 4-éthoxycarbonylbenzènesulfonamide sont portées à reflux du 100 ml de toluène anhydre en présence de 3,45 ml de triéthylamine pendant 20 heures. Après refroidissement, la phase toluénique est lavée à l'eau puis avec une solution de NaHCO₃ puis à nouveau à l'eau. L'ester ainsi obtenu est purifié par chromatographie sur colonne de silice en utilisantcomme éluant un mélange dichlorométhane/acétone (98/2). Le produit attendu est alors obtenu par saponification de l'ester dans un mélange soude/éthanol et est purifié par chromatographie sur colonne de silice en utilisant comme éluant un mélange dichlorométhane/méthanol (90/10).

### Préparation M : Acide 4-[(3,5-ditertbutyl-4-hydroxybenzyl)carbonylaminosulfonyl]benzoïque

Le produit attendu a été préparé selon le procédé décrit dans la préparation J.

| Microanalyse élémentaire : | | | | |
|---|---|---|---|---|
| | C % | H % | N % | S % |
| calculé | 61,73 | 6,53 | 3,13 | 7,16 |
| trouvé | 61,37 | 6,60 | 3,35 | 7,07 |

### Préparation N : Acide 4-[4-(2,6-diméthyl-3,5-diéthoxycarbonylpyridin-4-yl)benzoylaminosulfonyl]benzoïque

Le produit attendu a été préparé selon le procédé décrit dans la préparation J en utilisant le 1-(3-diméthylaminopropyl)-3-éthylcarbodiimide comme agent de couplage.

### Préparation O : Acide 4-{4-[2-(1-oxa-2-oxo-3,8-diazaspiro[4.5]déc-8-yl)éthyl]benzoylaminosulfonyl}benzoïque

Le produit attendu a été préparé selon le procédé décrit dans la préparation N à partir du composé décrit dans la préparation C et de 4-éthoxycarbonylbenzènesulfonamide.

### Préparation P : Acide 4-{4-chloro-3-[(3,5-ditertbutyl-4-hydroxybenzyl)carbonylaminosulfonyl]benzoylaminosulfonyl}benzoïque

Le produit attendu a été obtenu par couplage, selon le procédé décrit dans la préparation J, de l'acide 4-hydroxy-3,5-ditertbutylphénylacétique avec le 4-chloro-3-sulfamoylbenzoate d'éthyle, saponification puis à nouveau couplage du composé obtenu avec le 4-éthoxycarbonylbenzènesulfonamide.

### Préparation Q : Acide 4-[3,5-ditertbutyl-4-(3,5-ditertbutyl-4-hydroxybenzoyloxy)benzoylaminosulfonyl]benzoïque

Le produit attendu a été obtenu selon le procédé décrit dans la préparation J.

| Microanalyse élémentaire : | | | | |
|---|---|---|---|---|
| | C % | H % | N % | S % |
| calculé | 66,74 | 7,11 | 2,10 | 4,82 |
| trouvé | 66,38 | 7,48 | 2,23 | 5,20 |

### Préparation R : Acide 4-{[4-méthoxy-3-(3-éthoxy-2-hydroxypropoxy)benzoyl]aminosulfonyl}benzoïque

Le produit attendu a été préparé selon le procédé décrit dans la préparation N à partir d'acide 4-méthoxy-3-(2,3-époxypropoxy)benzoïque et de 4-éthoxycarbonylbenzènesulfonamide.
- Spectre de masse :: Ionisation chimique/NH₃
[M+H]⁺ : m/z = 454 (masse théorique M = 453)

### Préparation S : Acide 4-[(3,5-ditertbutyl-4-hydroxy)benzoylaminosulfonyl]benzoïque

Le composé a été obtenu selon le procédé décrit dans la préparation J.

### Préparation T : Acide 4-{4-[3,5-ditertbutyl-4-(éthoxyméthoxy)benzyloxy]benzoylaminosulfonyl}benzoïque

Le composé a été préparé selon le procédé décrit dans la préparation J.

### Préparation U : Acide 4-[(3,5-ditertbutyl-4-hydroxyphénylthio)acétylaminosulfonyl]benzoïque

Le composé a été préparé selon le procédé décrit dans la préparation J.

### Préparation V : Acide 4-{[3-(3,5-ditertbutyl-4-éthoxyméthoxyphényl)(trans)acryloyl]aminosulfonyl}benzoïque

Le composé a été préparé selon le procédé décrit dans la préparation J.

| Microanalyse élémentaire : | | | | |
|---|---|---|---|---|
| | C % | H % | N % | S % |
| calculé | 62,65 | 6,82 | 2,71 | 6,19 |
| trouvé | 62,46 | 7,25 | 2,93 | 6,56 |

### Exemple 1 : 4-(4-Chlorobenzoylaminosulfonyl)benzoyl-(S)Val-(S)Abo-(R,S)Val-CF₃

### Stade A : Boc-(S)Val-(S)Abo-OCH₂CH₃

En utilisant la technique de couplage peptidique décrite par W. KONIG et R. GEIGER (Chem. Ber., 103, 788, 1970), on fait réagir 100 mmoles de Boc-(S)Val-OH et 100 mmoles de 3-(S)-carbéthoxy-2-azabicyclo[2.2.2]octane. Le produit attendu est alors obtenu après purification sur gel de silice en utilisant comme éluant un mélange dichlorométhane/éthanol (97/3).
Rendement : 80 %

| Microanalyse élémentaire : | | | |
|---|---|---|---|
| | C % | H % | N % |
| calculé | 62,80 | 8,96 | 7,32 |
| trouvé | 62,42 | 8,78 | 7,26 |

### Stade B : Boc-(S)Val-(S)Abo-OH

Le produit attendu est obtenu par saponification du composé obtenu au stade précédent dans une solution contenant 150 ml d'éthanol et 100 ml de soude 1N pendant 72 heures. Après évaporation de l'éthanol et addition de 200 ml d'eau, lavage de la phase aqueuse à l'éther et acidification de celle-ci par addition d'acide citrique, le composé précipite et est lavé à l'eau puis à l'éther.
Rendement : 80 %
Point de fusion : 186°C

### Stade C : N-(Boc-(S)Val-(S)Abo)-1-isopropyl-2-hydroxy-3,3,3-trifluoropropylamine

21 mmoles du composé obtenu au stade précédent et 21 mmoles de chlorhydrate de 1-isopropyl-2-hydroxy-3,3,3-trifluoropropylamine (décrit dans J. Med. Chem., 33, 394-407, 1990) sont couplées selon la technique de couplage peptidique décrite au stade A. Le produit attendu est obtenu après purification sur gel de silice en utilisant comme éluant un mélange dichlorométhane/éthanol (97,5/2,5).
Rendement : 96 %

| Microanalyse élémentaire : | | | |
|---|---|---|---|
| | C % | H % | N % |
| calculé | 56,79 | 7,94 | 8,28 |
| trouvé | 56,65 | 7,74 | 8,48 |

### Stade D : Boc-(S)Val-(S)Abo-(R,S)Val-CF₃

5 mmoles du composé obtenu au stade précédent sont dissoutes dans 30 ml de diméthylsulfoxyde et 30 ml d'anhydrique acétique. Après 20 heures d'agitation à température ambiante et addition de 75 ml d'eau, l'agitation est maintenue une heure supplémentaire. L'ensemble est versé sur 150 ml d'eau et alcalinisé par addition de bicarbonate de sodium. Après extraction par du dichlorométhane, lavage des phases organiques à l'eau, séchage et évaporation, le produit attendu est obtenu après purification sur colonne de silice en utilisant comme éluant un mélange dichlorométhane/acétone (90/10).
Rendement : 65 %

| Microanalyse élémentaire : | | | |
|---|---|---|---|
| | C % | H % | N % |
| calculé | 57,02 | 7,58 | 8,31 |
| trouvé | 57,69 | 7,58 | 8,12 |

### Stade E : (S)Val-(S)Abo-(R,S)Val-CF₃, chlorhydrate

7 mmoles du produit obtenu au stade précédent sont déprotégées par agitation, à température ambiante, dans 100 ml d'une solution 3N d'acide chlorhydrique dans l'acétate d'éthyle. Après évaporation du solvant et addition de 100 ml d'éther, le gel ainsi obtenu est séché et conduit au produit attendu.
Rendement : 90 %

### Stade F : 4-(4-Chlorobenzoylaminosulfonyl)benzoyl-(S)Val-(S)-(R,S)Val-CF₃

1 mmole du produit obtenu au stade précédent et 1 mmole d'acide 4-(4-chlorobenzoylaminosulfonyl)benzoïque décrit dans la préparation A sont couplées selon la technique décrite par B. CASTRO et Coll. (Tet. Lett., 14, 1219-1222, 1975). Après évaporation du diméthylformamide, le résidu est dissous dans 100 ml d'acétate d'éthyle, lavé par 50 ml d'une solution saturée de bicarbonate de soude puis par de l'eau. Après séchage et évaporation, le produit attendu est obtenu par chromatographie sur gel de silice en utilisant comme éluant un mélange dichlorométhane/méthanol (90/10).
Rendement : 60 %
- Spectre de masse :: FAB - [M+H]⁺ : m/_{z} = 727
(masse théorique - ³⁵Cl - M = 726)

Les exemples 2 à 20 ont été obtenus en utilisant le même procédé que celui décrit pour l'exemple 1 en utilisant les produits de départ correspondants.

### Exemple 2 : 4-{[3-(3-Azabicyclo[3.3.0]octane)uréïdo]sulfonyl}benzoyl-(S)Val-(S)Abo-(R,S)Val-CF₃

- Spectre de masse :: FAB - [M+H]⁺ : m/_{z} = 741
(masse théorique M = 740)

### Exemple 3 : 4-(4-Chlorophénylsulfonylaminocarbonyl)benzoyl-(S)Val-(S)Abo-(R,S)Val-CF₃

- Spectre de masse :: FAB - [M-H]⁻ : m/_{z} = 725
(masse théorique - ³⁵Cl - M = 726)

### Exemple 4 : 4-(4-Chlorophénylsulfonylaminocarbonyl)benzoyl-(S)Val-(S)Abo-Val-CF₃, isomère α

- Spectre de masse :: FAB - [M-H]⁻ : m/_{z} = 725
(masse théorique - ³⁵Cl - M = 726)

### Exemple 5 : 4-(4-Chlorophénylsulfonylaminocarbonyl)benzoyl-(S)Val-(S)Abo-Val-CF₃, isomère β

- Spectre de masse :: FAB - [M-H]⁻ : m/_{z} = 725
(masse théorique - ³⁵Cl - M = 726)

Les isomères α et β du composé de l'exemple 3 sont séparés par chromatographie liquide préparative sur phase inverse C₁₈ en utilisant comme éluant un mélange eau-0,05M NaHCO₃/acétonitrile (80/20). Les éluats sont concentrés, acidifiés par de l'acide citrique à 10 %, extraits au dichlorométhane, lavés à l'eau, séchés et évaporés. Les isomères α et β sont ainsi nommés par leur ordre de sortie de colonne.

### Exemple 6 : 4-(4-Chlorophénylsulfonylaminocarbonyl)benzoyl-(S)Val-(2S,3aS, 7aS)Phi-(R,S)Val-CF₃

- Spectre de masse :: FAB - [M-H]⁻ : m/_{z} = 739
(masse théorique - ³⁵Cl - M = 740)

### Exemple 7 : 4-(4-Chlorophénylsulfonylaminocarbonyl)benzoyl-(S)Val-(2S,3aS, 7aS)Phi-Val-CF₃, isomère α

- Spectre de masse :: FAB - [M-H]⁻ : m/_{z} = 739
(masse théorique - ³⁵Cl - M = 740)

### Exemple 8 : 4-(4-Chlorophénylsulfonylaminocarbonyl)benzoyl-(S)Val-(2S,3aS, 7aS)Phi-Val-CF₃, isomère β

- Spectre de masse :: FAB - [M-H]⁻ : m/_{z} = 739
(masse théorique - ³⁵Cl - M = 740)

Les isomères α et β du composé de l'exemple 6 sont séparés selon la même technique que celle décrite pour les exemples 4 et 5. Le solvant d'élution est un mélange : eau-0,05M NaHCO₃/acétonitrile : 75/25.

### Exemple 9 : 4-[(R,S)-2-Oxotétrahydrothiophèn-3-yl)aminosulfonyl]benzoyl-(S)Val-(S)Abo-(R,S)Val-CF₃

- Spectre de masse :: FAB - [M-H]⁻ : m/_{z} = 687
(masse théorique - M = 688)

### Exemple 10 : 4-[(2-Oxotétrahydrothiophèn-3-yl)aminosulfonyl]benzoyl-(S)Val-(S)Abo-Val-CF₃, mélange d'isomères α

- Spectre de masse :: FAB - [M-H]⁻ : m/_{z} = 687
(masse théorique - M = 688)

### Exemple 11 : 4-[(2-Oxotétrahydrothiophen-3-yl)aminosulfonyl]benzoyl-(S)Val-(S)Abo-Val-CF₃, mélange d'isomères β

- Spectre de masse :: FAB - [M-H]⁻ : m/_{z} = 687
(masse théorique - M = 688)

Les produits attendus sont obtenus en utilisant au stade F le produit décrit dans la préparation B. Le composé de l'exemple 9 est un mélange de 4 isomères dont on sépare deux couples selon la technique décrite pour les exemples 4 et 5. Le solvant d'élution est un mélange : eau-0,05M NaHCO₃/acétonitrile : 65/35.

### Exemple 12 : 4-[2-(1-Oxa-2-oxo-3,8-diazaspiro[4.5]déc-8-yl)éthyl]benzoyl-(S)Val-(S)Abo-(R,S)Val-CF₃

Le produit attendu est obtenu en utilisant au stade F le produit décrit dans la préparation C.
- Spectre de masse :: FAB - [M+H]⁺ : m/_{z} = 692
(masse théorique - M = 691)

### Exemple 13 : 4-[4-[2-(1-Oxa-2-oxo-3,8-diazaspiro[4.5]déc-8-yl)éthyl]benzoylaminosulfonyl]benzoyl-(S)Val-(S)Abo-(R,S)Val-CF₃

### Exemple 14 : 4-[(4-Hydroxy-3,5-ditertbutylphénylacétyl)aminosulfonyl]benzoyl-(S)Val-(S)Abo-(R,S)Val-

**CF**_{**3**}

| Microanalyse élémentaire : | | | | |
|---|---|---|---|---|
| | C % | H % | N % | S % |
| calculé | 60,42 | 6,88 | 6,71 | 3,84 |
| trouvé | 60,96 | 7,20 | 7,25 | 3,38 |

### Exemple 15 : 4-[2-(4-Hydroxy-3,5-ditertbutylphénylthio)éthyl]benzoyl-(S)Val-(S)Abo-(R,S)Val-CF₃

Le produit attendu est obtenu en utilisant au stade F le produit décrit dans la préparation D.

| Microanalyse élémentaire : | | | | |
|---|---|---|---|---|
| | C % | H % | N % | S % |
| calculé | 65,18 | 7,55 | 5,43 | 4,14 |
| trouvé | 65,28 | 7,77 | 5,23 | 4,03 |

### Exemple 16 : 4-[4-[2-(4-Hydroxy-3,5-ditertbutylphénylthio)éthyl]benzoylaminosulfonyl]benzoyl-(S)Val-(S)Abo-(R,S)Val-CF₃

Le produit attendu est obtenu en utilisant au stade F le produit décrit dans la préparation E.

### Exemple 17 : 4-[(2,5,7,8-Tétraméthyl-6-hydroxychroman-2-yl)carbonylaminosulfonyl]benzoyl-(S)Val-(S)Abo-(R,S)Val-CF₃

Le produit attendu est obtenu en utilisant au stade F le produit décrit dans la préparation F.

| Microanalyse élémentaire : | | | | |
|---|---|---|---|---|
| | C % | H % | N % | S % |
| calculé | 58,52 | 6,26 | 6,82 | 3,91 |
| trouvé | 58,50 | 6,51 | 7,04 | 3,65 |

### Exemple 18 : 4-[S-Tertbutyl-N-acétyl-(S)cystéinylaminosulfonyl]benzoyl-(S)Val-(S)Abo-(R,S)Val-CF₃

Le produit attendu est obtenu en utilisant au stade F le produit décrit dans la préparation G.

| Microanalyse élémentaire : | | | | |
|---|---|---|---|---|
| | C % | H % | N % | S % |
| calculé | 53,22 | 6,38 | 8,87 | 8,12 |
| trouvé | 53,22 | 6,28 | 8,57 | 8,38 |

### Exemple 19 : 4-[2-[(1,3-Thiazolidin-3-yl)carbonyl]benzoylaminosulfonyl]benzoyl-(S)Val-(S)Abo-(R,S)Val-CF₃

Le produit attendu est obtenu en utilisant au stade F le produit décrit dans la préparation H.

### Exemple 20 : 4-[(4-Hydroxy-2-méthyl-1,1-dioxo-1,2-benzothiazin-3-yl)carbonyl]benzoyl-(S)Val-(S)Abo-(R,S)Val-CF₃

Le produit attendu est obtenu en utilisant au stade F le produit décrit dans la préparation I.

| Microanalyse élémentaire : | | | | |
|---|---|---|---|---|
| | C % | H % | N % | S % |
| calculé | 57,90 | 5,53 | 7,50 | 4,29 |
| trouvé | 57,53 | 5,53 | 7,48 | 4,30 |

### Exemple 21 : 4-[(4-Hydroxy-1,1-dioxo-(2H)-1,2-benzothiazin-3-yl)carbonyl]benzoyl-(S)Val-(S)Abo-(R,S)Val-CF₃

Le produit attendu est obtenu en utilisant au stade F le produit synthétisé par saponification du composé décrit dans la préparation I.
- Spectre de masse :: FAB - [M+H]⁺ : m/_{z} = 733
(masse théorique - M = 732)

### Exemple 22 : 4-(4-Chlorophénylsulfonylaminocarbonyl)benzoyl-(S)Val-(1S,3S,4R)Abh-(R,S)Val-CF₃

### Stade A : N-[(1S,3S,4R)Abh]-2-hydroxy-1-isopropyl-3,3,3-trifluoropropylamine, chlorhydrate

21 mmoles de Boc-(1S,3S,4R)Abh-OH sont couplées, selon la technique de couplage peptidique de W. KONIG et R. GEIGER, avec 21 mmoles de chlorhydrate de 2-hydroxy-1-isopropyl-3,3,3-trifluoropropylamine. Le produit attendu est obtenu après purification sur colonne de silice en utilisant comme éluant un mélange dichlorométhane/méthanol (97/3).

### Stade B : N-[Boc-(S)Val-(1S,3S,4R)Abh]-2-hydroxy-1-isopropyl-3,3,3-trifluoro-propylamine

Le produit attendu est obtenu par couplage de 12 mmoles du composé décrit au stade A avec 12 mmoles de Boc-(S)Val-OH selon la même technique de couplage peptidique et est purifié par chromatographie sur colonne de silice en utilisant comme solvant d'élution un mélange dichlorométhane/éthanol (95/5).

### Stade C : Boc-(S)Val-(1S,3S,4R)Abh-(R,S)Val-CF₃

Le produit attendu est obtenu par oxydation du composé décrit au stade B, selon le même procédé que celui décrit au stade D de l'exemple 1 et est purifié par chromatographie sur colonne de silice en utilisant comme éluant un mélange dichlorométhane/acétone (93/7).

### Stade D : (S)Val-(1S,3S,4R)Abh-(R,S)Val-CF₃, chlorhydrate

Le produit attendu est obtenu par déprotection du composé décrit au stade C selon la même technique que celle décrite au stade E de l'exemple 1.

### Stade E : 4-(4-Chlorophénylsulfonylaminocarbonyl)benzoyl-(S)Val-(1S,3S,4R)Abh-(R,S)Val-CF₃

Le produit attendu est obtenu selon le procédé décrit au stade F de l'exemple 1 en faisant réagir le composé décrit au stade D avec l'acide 4-(4-chlorophénylsulfonylaminocarbonyl)benzoïque et est purifié par chromatographie sur colonne de silice en utilisant comme éluant un mélange dichlorométhane/méthanol (90/10).
- Spectre de masse :: FAB - [M+H]⁺ : m/_{z} = 713
(masse théorique - ³⁵Cl - M = 712)

### Exemple 23 : 4-{2-[(1,3-Thiazolidin-3-yl)carbonyl]benzoylaminosulfonyl}benzoyl-(S)Val-(1S,3S,4R)Abh-(R,S)Val-CF₃

Le produit attendu est obtenu selon le procédé décrit dans l'exemple 22 en utilisant au stade E le produit décrit dans la préparation H.
- Spectre de masse :: FAB - [M+H]⁺ : m/_{z} = 794
(masse théorique - M = 793)

Les exemples 24 à 39 ont été obtenus selon le même procédé que celui décrit dans l'exemple 1 en utilisant les produits de départ correspondants.

### Exemple 24 : 4-[(Dicyclopropylméthyl)carbonylaminosulfonyl]benzoyl-(S)Val-(S)Abo-(R,S)Val-CF₃

Le produit attendu est obtenu en utilisant au stade F le produit décrit dans la préparation J.

| Microanalyse élémentaire : | | | | |
|---|---|---|---|---|
| | C % | H % | N % | S % |
| calculé | 57,45 | 6,38 | 7,88 | 4,51 |
| trouvé | 57,57 | 6,75 | 7,91 | 4,32 |

### Exemple 25 : 4-(4-Chlorophényluréïdosulfonyl)benzoyl-(S)Val-(S)Abo-(R,S)Val-CF₃

Le produit attendu est obtenu en utilisant au stade F le produit décrit dans la préparation K.

| Microanalyse élémentaire : | | | | | |
|---|---|---|---|---|---|
| | C % | H % | N % | Cl % | S % |
| calculé | 53,40 | 5,30 | 9,44 | 4,78 | 4,32 |
| trouvé | 53,86 | 5,28 | 9,55 | 4,22 | 4,77 |

### Exemple 26 : 4-(4-Chlorophényluréïdosulfonyl)benzoyl-(S)Val-(2S,3aS,7aS)Phi-(R,S)Val-CF₃

Le produit attendu est obtenu en utilisant au stade F le produit décrit dans la préparation K.

| Microanalyse élémentaire : | | | | | |
|---|---|---|---|---|---|
| | C % | H % | N % | Cl % | S % |
| calculé | 51,54 | 5,72 | 8,84 | 4,47 | 4,05 |
| trouvé | 51,74 | 5,85 | 8,53 | 4,09 | 3,65 |

### Exemple 27 : 4-[(Dicyclopropylméthyl)carbonylaminosulfonyl]benzoyl-(S)Val-(2S,3aS,7aS)Phi-(R,S)Val-CF₃

Le produit attendu est obtenu en utilisant au stade F le produit décrit dans la préparation J.

| Microanalyse élémentaire : | | | | |
|---|---|---|---|---|
| | C % | H % | N % | S % |
| calculé | 58,00 | 6,54 | 7,73 | 4,42 |
| trouvé | 58,20 | 6,91 | 7,95 | 4,38 |

### Exemple 28 : 4-[(Adamant-1-yl)carbonylaminosulfonyl]benzoyl-(S)Val-(S)Abo-(R,S)Val-CF₃

Le produit attendu est obtenu en utilisant au stade F le produit décrit dans la préparation L.

| Microanalyse élémentaire : | | | | |
|---|---|---|---|---|
| | C % | H % | N % | S % |
| calculé | 59,18 | 6,58 | 7,46 | 4,26 |
| trouvé | 59,18 | 7,04 | 8,05 | 4,10 |

### Exemple 29 : 4-[(3,5-Ditertbutyl-4-hydroxybenzyl)carbonylaminosulfonyl]benzoyl-(S)Val-(2S,3aS,7aS)Phi-(R,S)Val-CF₃

Le produit attendu est obtenu en utilisant au stade F le produit décrit dans la préparation M.

| Microanalyse élémentaire : | | | | |
|---|---|---|---|---|
| | C % | H % | N % | S % |
| calculé | 60,83 | 7,00 | 6,60 | 3,78 |
| trouvé | 60,67 | 7,33 | 6,97 | 3,85 |

### Exemple 30 : 4-[4-(2,6-Diméthyl-3,5-diéthoxycarbonyl-1,4-dihydropyridin-4-yl)benzoylaminosulfonyl]benzoyl-(S)Val-(S)Abo-(R,S)Val-CF₃

Le produit attendu est obtenu en utilisant au stade F le produit décrit dans la préparation N.

| Microanalyse élémentaire : | | | | |
|---|---|---|---|---|
| | C % | H % | N % | S % |
| calculé | 58,53 | 5,98 | 7,42 | 3,40 |
| trouvé | 57,86 | 5,94 | 7,12 | 3,30 |

### Exemple 31 : 4-{4-[2-(3,5-Ditertbutyl-4-hydroxyphénylthio)éthyl]benzoylaminosulfonyl}benzoyl-(S)Val-(2S,3aS,7aS)Phi-(R,S)Val-CF₃

Le produit attendu est obtenu en utilisant au stade F le produit décrit dans la préparation E.

| Microanalyse élémentaire : | | | | |
|---|---|---|---|---|
| | C % | H % | N % | S % |
| calculé | 61,84 | 6,75 | 5,77 | 6,60 |
| trouvé | 61,73 | 7,04 | 5,49 | 5,49 |

### Exemple 32 : 4-{4-[2-(1-Oxa-2-oxo-3,8-diazaspiro[4.5]dec-8-yl)éthyl]benzoylaminosulfonyl}benzoyl-(S)Val-(2S,3aS,7aS)Phi-(R,S)Val-CF₃

Le produit attendu est obtenu en utilisant au stade F le produit décrit dans la préparation O.
- Spectre de masse :: FAB-[M]⁺ : m/z = 888 (masse théorique : M = 888)

### Exemple 33 : 4-{[4-Chloro-3-[(3,5-ditertbutyl-4-hydroxybenzyl)carbonylaminosulfonyl]benzoyl]aminosulfonyl}benzoyl-(S)Val-(2S,3aS,7aS)Phi-(R,S)Val-CF₃

Le produit attendu est obtenu en utilisant au stade F le produit décrit dans la préparation P.

| Microanalyse élémentaire : | | | | |
|---|---|---|---|---|
| | C % | H % | N % | S % |
| calculé | 56,30 | 5,95 | 6,57 | 6,01 |
| trouvé | 56,16 | 6,45 | 6,67 | 6,08 |

### Exemple 34 : 4-{[3,5-Ditertbutyl-4-(3,5-ditertbutyl-4-hydroxybenzoyloxy)benzoyl]aminosulfonyl}benzoyl-(S)Val-(2S,3aS,7aS)Phi-(R,S)Val-CF₃

Le produit attendu est obtenu en utilisant au stade F le produit décrit dans la préparation Q.

| Microanalyse élémentaire : | | | | |
|---|---|---|---|---|
| | C % | H % | N % | S % |
| calculé | 64,14 | 7,27 | 5,25 | 3,00 |
| trouvé | 63,81 | 7,32 | 5,39 | 3,73 |

### Exemple 35 : 4-{[4-Méthoxy-3-(3-éthoxy-2-hydroxypropoxy)benzoyl]aminosulfonyl}benzoyl-(S)Val-(2S,3aS,7aS)Phi-(R,S)Val-CF₃

Le produit attendu est obtenu en utilisant au stade F le produit décrit dans la préparation R.
- Spectre de masse :: FAB-[M-H]⁻ : m/z = 853 (masse théorique : M = 854)

### Exemple 36 : 4-[(3,5-Ditertbutyl-4-hydroxybenzoyl)aminosulfonyl]benzoyl-(S)Val-(2S,3aS,7aS)Phi-(R,S)Val-CF₃

Le produit attendu est obtenu en utilisant au stade F le produit décrit dans la préparation S.

| Microanalyse élémentaire : | | | | |
|---|---|---|---|---|
| | C % | H % | N % | S % |
| calculé | 60,42 | 6,88 | 6,71 | 3,84 |
| trouvé | 60,84 | 7,28 | 6,92 | 4,21 |

### Exemple 37 : 4-{4-[3,5-Ditertbutyl-4-(éthoxyméthoxy)benzyloxy]benzoyl aminosulfonyl}benzoyl-(S)Val-(2S,3aS,7aS)Phi-(R,S)Val-CF₃

Le produit attendu est obtenu en utilisant au stade F le produit décrit dans la préparation T.

| Microanalyse élémentaire : | | | | |
|---|---|---|---|---|
| | C % | H % | N % | S % |
| calculé | 62,51 | 6,96 | 5,61 | 3,21 |
| trouvé | 62,17 | 7,37 | 5,74 | 3,23 |

### Exemple 38 : 4-[(3,5-Ditertbutyl-4-hydroxyphénylthio)acétylaminosulfonyl]benzoyl-(S)Val-(2S,3aS,7aS)Phi-(R,S)Val-CF₃

Le produit attendu est obtenu en utilisant au stade F le produit décrit dans la préparation U.

| Microanalyse élémentaire : | | | | |
|---|---|---|---|---|
| | C % | H % | N % | S % |
| calculé | 58,62 | 6,75 | 6,36 | 7,28 |
| trouvé | 58,79 | 7,12 | 6,02 | 7,93 |

### Exemple 39 : 4-{[3-(3,5-Ditertbutyl-4-éthoxyméthoxyphényl)(trans)acryloyl]aminosulfonyl}benzoyl-(S)Val-(2S,3aS,7aS)Phi-(R,S)Val-CF₃

Le produit attendu est obtenu en utilisant au stade F le produit décrit dans la préparation V.

| Microanalyse élémentaire : | | | | |
|---|---|---|---|---|
| | C % | H % | N % | S % |
| calculé | 61,42 | 7,13 | 6,10 | 3,49 |
| trouvé | 61,22 | 7,10 | 6,10 | 3,43 |

### Exemple 40 : 4-{[3-(3,5-Ditertbutyl-4-hydroxyphényl)(trans)acryloyl]aminosulfonyl}benzoyl-(S)Val-(2S,3aS,7aS)Phi-(R,S)Val-CF₃

Le produit attendu a été obtenu après hydrolyse en milieu dioxanechlorhydrique du composé décrit dans l'exemple 39 et purification par chromatographie sur colonne de silice en utilisant comme éluant un mélange dichlorométhane/méthanol (95/5).

| Microanalyse élémentaire : | | | | |
|---|---|---|---|---|
| | C % | H % | N % | S % |
| calculé | 61,38 | 7,22 | 6,42 | 4,18 |
| trouvé | 61,12 | 6,91 | 6,51 | 3,72 |

### Exemple 41 : 4-(4-Chlorophénylsulfonylaminocarbonyl)benzoyl-[N-(3,5-ditertbutyl-4-hydroxyphényl)acétyl](S)Lys-(2S,3aS,7aS)Phi-(R,S)Val-CF₃

### Stade A : 4-(4-Chlorophénylsulfonylaminocarbonyl)benzoyl-(S)Lys-OCH₃

L'acide 4-(4-chlorophénylsulfonylaminocarbonyl)benzoïque est couplé avec l'ester méthylique de (S)Lysine protégé par un groupement tertbutoxy sur sa fonction amine latérale en utilisant comme réactif de couplage le BOP en présence de diéthylamine dans du diméthylformamide. Le produit attendu est alors déprotégé par hydrolyse acide à l'aide d'acide bromhydrique en milieu acide acétique et est purifié par chromatographie sur colonne de silice en utilisant comme éluant un mélange dichlorométhane/méthanol (60/40).

### Stade B : 4-(4-Chlorophénylsulfonylaminocarbonyl)benzoyl-[N-(3,5-ditertbutyl-4-hydroxyphényl)acétyl](S)Lys-OH

Le composé obtenu au stade précédent est couplé selon la technique de W. KONIG et R. GEIGER (Chem. Ber., 103, 788, 1970) avec l'acide 3,5-ditertbutyl-4-hydroxyphénylacétique. Le produit attendu sous forme d'ester méthylique est purifié par chromatographie sur colonne de silice en utilisant comme éluant un mélange dichlorométhane/méthanol (90/10) et est ensuite saponifié par un mélange soude/éthanol.

### Stade C : 4-(4-Chlorophénylsulfonylaminocarbonyl)benzoyl-[N-(3,5-ditertbutyl-4-hydroxyphényl)acétyl](S)Lys-(2S,3aS,7aS)Phi-(R,S)Val-CF₃

Le produit obtenu au stade précédent est couplé avec (S)Phi-(R,S)-Val-CF₃ selon la technique décrite au stade précédent et conduit au produit attendu qui est purifié par chromatographie sur colonne de silice en utilisant comme éluant un mélange dichlorométhane/méthanol (90/10).

| Microanalyse élémentaire : | | | | |
|---|---|---|---|---|
| | C % | H % | N % | S % |
| calculé | 60,26 | 6,44 | 6,89 | 3,15 |
| trouvé | 59,37 | 6,59 | 6,43 | 2,77 |

Les exemples 42 et 43 ont été obtenus selon le procédé décrit pour l'exemple 41.

### Exemple 42 : 4-(4-Chlorophénylsulfonylaminocarbonyl)benzoyl-[N-(3,5-ditertbutyl-4-hydroxyphénylthio)butyroyl](S)-Lys-(2S,3aS,7aS)Phi-(R,S)Val-CF₃

- Spectre de masse :: FAB : [M+H]⁺ : m/z = 1076 (masse théorique : M = 1075)

### Exemple 43 : 4-(4-Chlorophénylsulfonylaminocarbonyl)benzoyl-[S-(3,5-ditertbutyl-4-hydroxyphényl)](S)Cys-(2S,3aS,7aS)Phi-(R,S)Val-CF₃

- Spectre de masse :: FAB : [M+H]⁺ : m/z = 949 (masse théorique : M = 948)

### Exemple 44 :

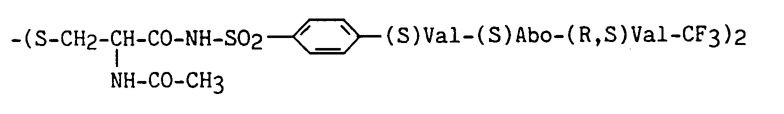

Le produit attendu a été obtenu par hydrolyse du composé de l'exemple 18 dans un mélange dioxane/eau (80/20) en présence d'hydrogène sulfuré et d'acétate mercurique. Après filtration, le filtrat est évaporé et conduit au produit attendu.

| Microanalyse élémentaire : | | | | |
|---|---|---|---|---|
| | C % | H % | N % | S % |
| calculé | 50,81 | 5,64 | 9,56 | 8,75 |
| trouvé | 51,42 | 5,58 | 9,50 | 8,32 |

### ETUDE PHARMACOLOGIQUE DES DÉRIVÉS DE L'INVENTION

### Exemple 45 : Activité inhibitrice de l'élastase leucocytaire humaine in vitro

La puissance des composés de l'invention est déterminée par le niveau d'inhibition de l'action de l'élastase leucocytaire humaine sur un substrat peptidique de bas poids moléculaire selon la technique décrite par B.M. ASHE et coll. (J. Biol. Chem., 256, 11603-11606, 1981). Cette activité est mesurée en suivant la cinétique d'hydrolyse du substrat qui entraîne la libération de paranitroaniline qui absorbe à une longueur d'onde de 410 nm.

### Réactif :

- Enzyme :: Elastase de sputum leucocytaire humain (Elastin Products Co.) solubilisée à 1000 uI/ml d'eau distilée, et congélée en aliquots de 50 µl à -20 °C.
- Substrat :: méthoxysuccinyl-L-alanyl-L-alanyl-prolyl-valine, paranitroanilide (Sigma chimie).
- Tampon: : Tris 0,1 M ; NaCl 0,5 M ; pH = 7,8.

### Matériel :

Spectrophotomètre thermostaté à 37 °C équipé d'un passeur de cuve.
Cuve de 1 ml en polystyrène.
Bain marie à sec réglé à 37 °C.

### Mode opératoire :

Dans une cuve on introduit :
- 970 µl de tampon
- 10 µl du produit à tester ou du solvant (concentré x 100)
- 10 µl d'élastase leucocytaire de sputum humain dilué au 1/10ème
- Agitation et incubation 15 mn à 37 °C
- La réaction est débutée par ajout de 10 µl de substrat.

La cuve est introduite dans le spéctrophotomètre, la densité optique est enregistrée en fonction du temps à 410 nm à 37 °C.
La vitesse initiale est mesurée pour chaque concentration de produit (ou témoin solvant) étudiée.
Des pourcentages d'inhibition par rapport au témoin solvant sont calculés :
Pourcentage d'inhibition = 100 x (vitesse témoin - vitesse du produit testé/vitesse témoin).
Les concentrations inhibitrices 50 (IC50) sont calculées à partir des pourcentages d'inhibition par régression linéaire simple.
Les résultats obtenus dans ce test sont rassemblés ci-dessous :

| Exemple | IC50 (nM) | Exemple | IC50 (nM) |
|---|---|---|---|
| 1 | 24 | 28 | 47 |
| 2 | 25 | 29 | 38 |
| 4 | 15 | 30 | 41 |
| 6 | 27 | 31 | 40 |
| 14 | 30 | 32 | 42 |
| 18 | 19 | 33 | 34 |
| 22 | 19 | 35 | 37 |
| 23 | 30 | 36 | 36 |
| 24 | 25 | 40 | 38 |
| 25 | 34 | 41 | 70 |
| 26 | 31 | 44 | 14 |
| 27 | 29 | | |

### Exemple 46 : Activité inhibitrice in vivo : modèle d'oedème aigu hémorragique chez le hamster

L'instillation trachéale chez le hamster d'une préparation purifiée d'élastase leucocytaire de sputum humain entraîne une hémorragie aigue qui peut être quantifiée par la mesure de la concentration d'hémoglobine dans le lavage broncho-alvéolaire 3 heures après l'instillation d'élastase.

L'étude est effectuée chez des hamsters mâles de 120 à 140 g. (Syrian Golden, n = 10 par lot). Les animaux sont anesthésiés par du pentobarbital à la dose de 40 mg/kg par voie intrapéritonéale.

Les hamsters sont anesthésiés et la trachée est exposée chirurgicalement. Les produits à tester sont administrés à l'aide d'une aiguille directement dans la trachée dans un volume 0,1 ml à la dose de 10 µg. L'élastase leucocytaire de sputum humain est administrée 3 heures après l'administration du produit, par voie intratrachéale à la dose 50 unités par animal sous un volume de 0,2 ml.

Les animaux sont sacrifiés à l'aide d'une dose léthale de pentobarbital, 3 heures après l'instillation de l'élastase, et un lavage broncho-alvéolaire au sérum physiologique est effectué. Le degré d'hémorragie est quantifié par méthode colorimétrique permettant le dosage de la concentration d'hémoglobine (Boehringer test-combination hémoglobine).

Les résultats sont exprimés en pourcentage d'inhibition de l'hémorragie.

Les composés de la présente invention sont des inhibiteurs effectifs de l'élastase leucocytaire humaine qui préviennent ou diminuent l'hémorragie induite par l'instillation intratrachéale d'élastase leucocytaire humaine. Les résultats obtenus dans ce test sont rassemblés ci-dessous :

| Exemple | Inhibition (%) | Exemple | Inhibition (%) |
|---|---|---|---|
| 1 | 67 | 27 | 38 |
| 2 | 57 | 28 | 57 |
| 4 | 70 | 29 | 63 |
| 6 | 77 | 30 | 56 |
| 14 | 28 | 31 | 20 |
| 18 | 38 | 32 | 42 |
| 22 | 65 | 33 | 37 |
| 23 | 61 | 35 | 47 |
| 24 | 46 | 36 | 53 |
| 25 | 63 | 40 | 40 |
| 26 | 64 | 41 | 41 |
| | | 44 | 44 |

### Exemple 47 : Etude de la péroxydation lipidique

L'étude a été pratiquée sur microsomes hépatiques de rat en présence de Fe³⁺ (100 µM) et d'ascorbate (100 µM). Le dosage du malondialdéhyde (MDA) est réalisé par la méthode à l'acide thiobarbiturique (spectrophotométrie λ = 532 nm) selon la technique décrite par N. PAYA et coll. (Biochem. Pharmacol., vol. 44, n° 2, p. 205-214, 1992).

Les produits de l'invention possèdent une activité inhibitrice comprise entre 10⁻⁶M et 10⁻⁵M sur la péroxydation lipidique dans le système étudié.

### Exemple 48 : Composition pharmaceutique

Formule de préparation pour 1000 comprimés dosés à 10 mg

| | |
|---|---|
| Composé de l'exemple 6 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 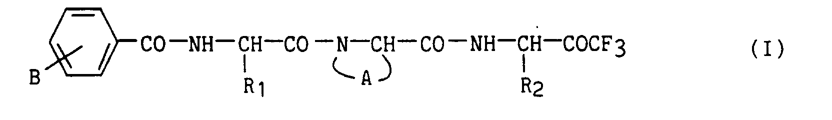 dans laquelle :
R₁ représente un radical alkyle (C₁-C₆) linéaire ou ramifié substitué ou non par un radical cycloalkyle (C₃-C₇), phényle, amino ou benzyloxycarbonylamino, (3,5-ditertbutyl-4-hydroxy)benzylcarbonylamino, (3,5-ditertbutyl-4-hydroxy)phénylthio ou 3-[(3,5-ditertbutyl-4-hydroxy)phénylthio]propylcarbonylamino,
R₂ représente un radical alkyle (C₁-C₆) linéaire ou ramifié,
A représente avec les atomes d'azote et de carbone auquel il est attaché un cycle 2-azabicyclo[2.2.2]octane, 2-azabicyclo[2.2.1] heptane, perhydroindole,
B représente l'un quelconque des radicaux suivants :
a 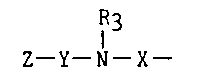 dans lequel :
R₃ représente un atome d'hydrogène ou un radical alkyle (C₁-C₆) linéaire ou ramifié substitué ou non par un radical phényle,
X, Y, différents, représentent CO ou SO₂,
Z représente
- un radical alkyle (C₁-C₆) linéaire ou ramifié substitué par un ou deux radicaux, identiques ou différents, cycloalkyle (C₃-C₇) ou trihalogénoalkyle (C₁-C₄),
- un radical adamant-1-yl,
- un radical phényle substitué ou non par un ou plusieurs, identiques ou différents, atomes d'halogène ou radicaux alkyle (C₁-C₆) linéaire ou ramifié, hydroxy, alkoxy (C₁-C₆) linéaire ou ramifié, trihalogénoalkyle (C₁-C₄), cyano, 1,4-dihydropyridin-4-yl (substitué ou non par un ou plusieurs radicaux, identiques ou différents, alkyle (C₁-C₆) linéaire ou ramifié ou alkoxycarbonyle (C₁-C₆) linéaire ou ramifié), (3,5-ditertbutyl-4-hydroxy) benzylcarbonylaminosulfonyl, (3,5-ditertbutyl-4-hydroxy) benzoyloxy, (3-éthoxy-2-hydroxy)propoxy, ou [3,5-ditertbutyl-4-(éthoxyméthoxy)]benzyloxy,
- ou, un radical alkényle (C₁-C₄) substitué par un radical [3,5-ditertbutyl-4-(éthoxyméthoxy)]phényle ou (3,5-ditertbutyl-4-hydroxy)phényle,
c 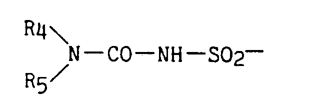 dans lequel :
R₅ représente un atome d'hydrogène,
R₄, représente phényle (substitué ou non par un ou plusieurs atomes d'halogène, radical alkyle (C₁-C₆) linéaire ou ramifié, alkoxy (C₁-C₆) linéaire ou ramifié, trihalogénoalkyle (C₁-C₄)), titué ou non par un radical alkyle (C₁-C₆) linéaire ou ramifié ou phénylalkyle (C₁-C₆) linéaire ou ramifié),
ou
forment avec l'atome d'azote auxquels ils sont attachés le cycle :
- 3-azabicyclo[3.3.0]octane,
f₁ 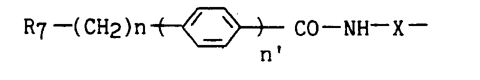
f₂ R₇―(CH₂)ₙ―
dans lesquels :
X est tel que défini précédemment,
n est égal à 0, 1, 2 ou 3,
n' est égal à 0 ou 1,
R₇ représente un radical :
. 3,5-ditertbutyl-4-hydroxyphényle,
. 3,5-ditertbutyl-4-hydroxy phénoxy,
. ou 3,5-ditertbutyl-4-hydroxy phénylthio,
h 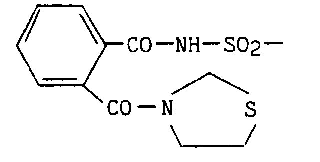
composés de formule (I) qui comprennent les hydrates de la fonction cétone COCF₃ correspondants,
leurs énantiomères, diastéréoisomères et épimères ainsi que leurs sels d'addition à un acide ou à une base pharmaceutiquement acceptable.

2. Composés de formule (I) selon la revendication 1 tels que B représente un radical 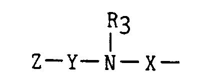 dans lequel :
R₃ représente un atome d'hydrogène ou un radical alkyle (C₁-C₆) linéaire ou ramifié substitué ou non par un radical phényle,
X ou Y, différents, représentent CO ou SO₂,
Z représente
- un radical alkyle (C₁-C₆) linéaire ou ramifié substitué par un ou deux radicaux, identiques ou différents, cycloalkyle (C₃-C₇) ou trihalogénoalkyle (C₁-C₄),
- un radical adamant-1-yl,
- un radical phényle substitué ou non par un ou plusieurs, identiques ou différents, atomes d'halogène ou radicaux alkyle (C₁-C₆) linéaire ou ramifié, hydroxy, alkoxy (C₁-C₆) linéaire ou ramifié, trihalogénoalkyle (C₁-C₄), cyano, 1,4-dihydropyridin-4-yl (substitué ou non par un ou plusieurs radicaux, identiques ou différents, alkyle (C₁-C₆) linéaire ou ramifié ou alkoxycarbonyle (C₁-C₆) linéaire ou ramifié), (3,5-ditertbutyl-4-hydroxy) benzylcarbonylaminosulfonyl, (3,5-ditertbutyl-4-hydroxy) benzoyloxy, (3-éthoxy-2-hydroxy)propoxy, ou [3,5-ditertbutyl-4-(éthoxyméthoxy)]benzyloxy,
- ou, un radical alkényle (C₁-C₄) substitué par un radical [3,5-ditertbutyl-4-(éthoxyméthoxy)]phényle ou (3,5-ditertbutyl-4-hydroxy)phényle,
leurs hydrates, énantiomères, diastéréoisomères et épimères ainsi que leurs sels d'addition à un acide ou à une base pharmaceutiquement acceptable.

3. Composés de formule (I) selon la revendication 1 tels que B représente un radical 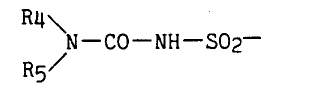 dans lequel :
R₅ représente un atome d'hydrogène,
R₄ représente phényle (substitué ou non par un ou plusieurs atomes d'halogène, radical alkyle (C₁-C₆) linéaire ou ramifié, alkoxy (C₁-C₆) linéaire ou ramifié, trihalogénoalkyle (C₁-C₄)),
ou forment avec l'atome d'azote auxquels ils sont attachés le cycle :
- 3-azabicyclo[3.3.0]octane,
leurs hydrates, énantiomères, diastéréoisomères et épimères ainsi que leurs sels d'addition à un acide ou à une base pharmaceutiquement acceptable.

4. Composés de formule (I) selon la revendication 1 tels que B représente un radical 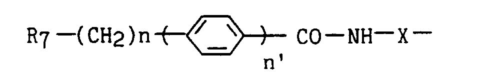 dans lequel :
X est tel que défini précédemment,
n est égal à 0, 1, 2 ou 3,
n' est égal à 0 ou 1,
R₇ représente un radical :
. 3,5-ditertbutyl-4-hydroxy phényle,
. 3,5-ditertbutyl-4-hydroxy phénoxy,
. ou 3,5-ditertbutyl-4-hydroxy phénylthio,
leurs hydrates, énantiomères, diastéréoisomères et épimères ainsi que leurs sels d'addition à un acide ou à une base pharmaceutiquement acceptable.

5. Composés de formule (I) selon la revendication 1 tels que A représente avec les atomes d'azote et de carbone auquel il est attaché un cycle 2-azabicyclo[2.2.2]octane, leurs hydrates, énantiomères, diastéréoisomères et épimères ainsi que leurs sels d'addition à un acide ou à une base pharmaceutiquement acceptable.

6. Composés de formule (I) selon la revendication 1 tels que A représente avec les atomes d'azote et de carbone auquel il est attaché un cycle perhydroindole, leurs hydrates, énantiomères, diastéréoisomères et épimères ainsi que leurs sels d'addition à un acide ou à une base pharmaceutiquement acceptable.

7. Composés de formule (I) selon l'une quelconque des revendications 5 ou 6 tels que B représente un radical 4-chlorobenzoylaminosulfonyl, leurs hydrates, énantiomères, diastéréoisomères et épimères ainsi que leurs sels d'addition à un acide ou à une base pharmaceutiquement acceptable.

8. Composés de formule (I) selon l'une quelconque des revendications 5 ou 6 tels que B représente un radical 4-chlorophénylsulfonylaminocarbonyl, leurs hydrates, énantiomères, diastéréoisomères et épimères ainsi que leurs sels d'addition à un acide ou à une base pharmaceutiquement acceptable.

9. Composé de formule (I) selon la revendication 1 qui est le 4-(4-chlorophénylsulfonylaminocarbonyl)benzoyl-(S)Val-(2S, 3aS, 7aS)Phi-(R,S) Val-CF₃, Phi représentant un résidu perhydroindole-2-carbonyle et Val un résidu valyle, ses hydrates, isomères ainsi que ses sels d'addition à un acide ou une base pharmaceutiquement acceptable.

10. Composé de formule (I) selon la revendication 1 qui est le 4-(4-chlorophénylsulfonylaminocarbonyl)benzoyl-(S)Val-(S)Abo-(R,S)Val-CF₃, Abo représentant un résidu 2-azabicyclo[2.2.2]octane-3-carbonyle et Val un résidu valyle, ses hydrates, isomères ainsi que ses sels d'addition à un acide ou une base pharmaceutiquement acceptable.

11. Procédé de préparation des dérivés de formule (I) selon la revendication 1 caractérisé en ce que l'on utilise, comme produit de départ un alcool de formule (II), dont on a éventuellement séparé les isomères par une technique classique de séparation : 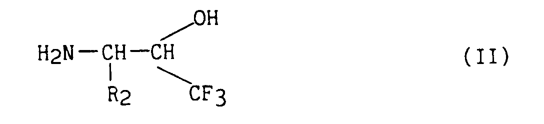 dans laquelle R₂ a la même signification que dans la formule (I), que l'on fait réagir :
a soit sur un amino-acide protégé de formule (III), dont on a éventuellement séparé les isomères selon une technique classique de séparation, par une technique classique de couplage peptidique : 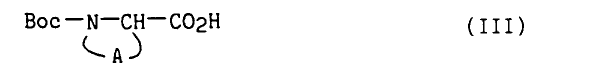 dans laquelle A a la même signification que dans la formule (I) et Boc représente un groupement butoxycarbonyle,
pour conduire au composé de formule (IV) : 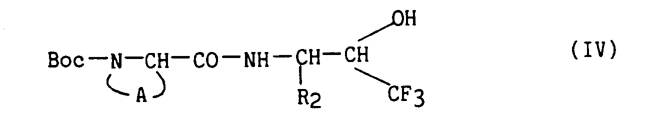 dans laquelle A, R₂ et Boc ont la même signification que précédemment,
composé de formule (IV) :
* ou bien que l'on déprotège par hydrolyse acide pour conduire au composé de formule (V) : 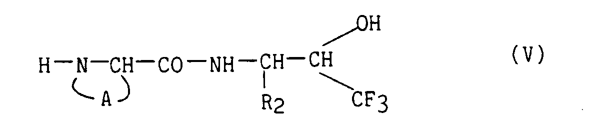 dans laquelle A et R₂ ont la même signification que précédemment,
sur lequel on fait réagir un amino-acide protégé de formule (VI), dont on a éventuellement séparé les isomères selon une technique classique de séparation,
en présence d'un agent de couplage classique de la synthèse peptidique : 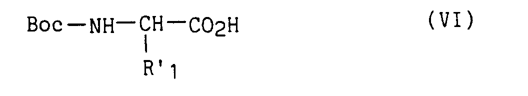 dans laquelle Boc représente un radical butoxycarbonyle et R'₁ représente un radical alkyle (C₁-C₆) linéaire ou ramifié substitué ou non par un radical cycloalkyle (C₃-C₇), phényle ou benzyloxycarbonylamino,
pour conduire au composé de formule (VII) : 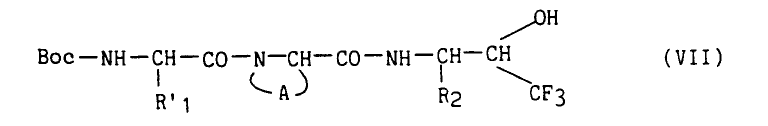 dans laquelle Boc, R'₁, A et R₂ ont la même signification que précédemment,
qui subit une oxydation pour conduire au composé de formule (VIII), dont on sépare éventuellement les isomères selon une technique classique de séparation, 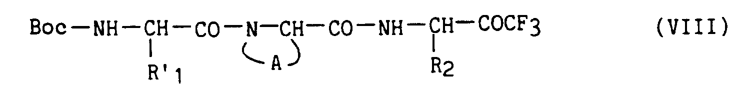 dans laquelle Boc, R'₁, A et R₂ ont la même signification que précédemment,
* ou bien qui subit une oxydation pour conduire au composé de formule (X) : 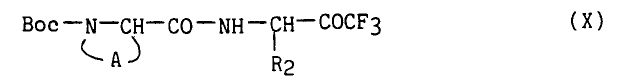 dans laquelle Boc, A et R₂ ont la même signification que précédemment,
que l'on déprotège en milieu acide pour conduire au composé de formule (XI) : 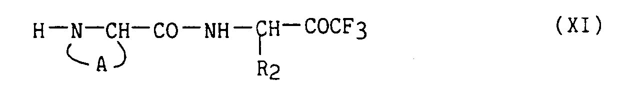 dans laquelle A et R₂ ont la même signification que précédemment,
que l'on fait réagir sur un amino-acide protégé de formule (VI) tel que défini précédemment,
pour conduire au composé de formule (VIII) défini précédemmment,
b soit sur un dipeptide protégé de formule (XII), obtenu par couplage classique de deux amino-acides sous forme racémique ou d'énantiomères purs, 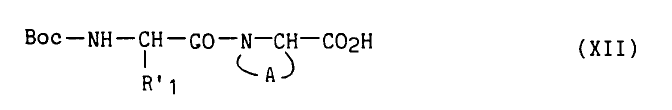 dans laquelle Boc, R'₁ et A ont la même signification que précédemment,
pour conduire au composé de formule (VII) défini précédemment,
qui subit une oxydation et conduit au composé de formule (VIII) défini précédemment,
composé de formule (VIII) que l'on déprotège en milieu acide,
pour conduire au composé de formule (IX), dont on sépare éventuellement les isomères selon une technique classique de séparation, 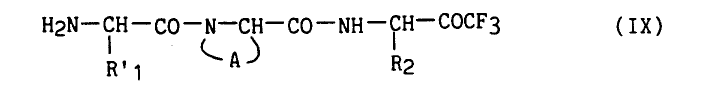 dans laquelle R'₁, A et R₂ ont la même signification que précédemment,
que l'on fait réagir sur un acide de formule (XIII), selon une technique classique de couplage peptidique, 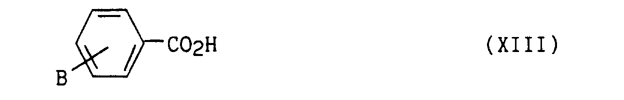 dans laquelle B a la même signification que dans la formule (I),
pour conduire au composé de formule (I/a), cas particulier des composés de formule (I), 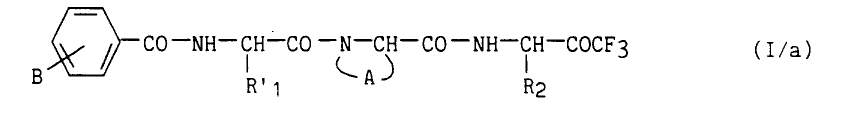 dans laquelle B, A, R'₁ et R₂ ont la même signification que précédemment,
qui, lorsque R'₁ représente un radical alkyle substitué par un groupement benzyloxycarbonylamino, est déprotégé, si on le souhaite, par hydrogénation catalytique,
pour conduire au composé de formule (I/b), cas particulier des composés de formule (I), 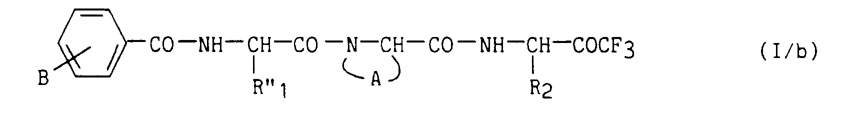 dans laquelle B, A et R₂ ont la même signification que dans la formule (I) et R''₁ représente un alkyle substitué par un radical amino,
composés de formule (I/a) et (I/b), que l'on purifie selon une technique classique de purification, dont on sépare, si on le désire, les isomères selon une technique classique de séparation, puis, si nécessaire, que l'on transforme en sel d'addition à un acide ou à une base pharmaceutiquement acceptable.

12. Compositions pharmaceutiques contenant comme principe actif au moins un composé selon l'une quelconque des revendications 1 à 10 seul ou en combinaison avec un ou plusieurs excipients ou véhicules inertes, non toxiques, pharmaceutiquement acceptables.

13. Compositions pharmaceutiques selon la revendication 12 contenant au moins un principe actif selon l'une quelconque des revendications 1 à 10 utilisé en tant qu'inhibiteur de l'élastase leucocytaire humaine à titre de traitement principal ou complémentaire des maladies dégénératives du tissu conjonctif comme l'emphysème pulmonaire, des troubles inflammatoires comme l'arthrite rhumatoïde, des pathologies pulmonaires du type du syndrome de détresse respiratoire aigu, de la fibrose kystique, de la bronchite chronique, des lésions tissulaires induites par le syndrome d'ischémie reperfusion et en tant que traitement topique sur la peau afin d'inhiber l'élastolyse.

## Claims

1. Compounds of formula (I) : 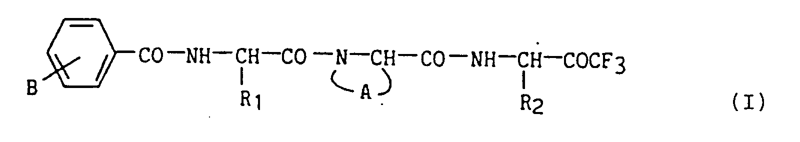 wherein :
R₁ represents a straight-chain or branched (C₁-C₆)-alkyl radical that is unsubstituted or substituted by a (C₃-C₇)-cycloalkyl, phenyl, amino or benzyloxycarbonylamino, (3,5-di-tert-butyl-4-hydroxy)-benzylcarbonylamino, (3,5-di-tert-butyl-4-hydroxy)-phenylthio or 3-[(3,5-di-tert-butyl-4-hydroxy)-phenylthio]propylcarbonylamino radical,
R₂ represents a straight-chain or branched (C₁-C₆)-alkyl radical,
A represents, with the nitrogen and carbon atoms to which it is attached, a 2-azabicyclo[2.2.2]octane, 2-azabicyclo[2.2.1]heptane or perhydroindole ring,
B represents any one of the following radicals :
a 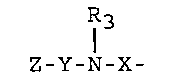 wherein :
R₃ represents a hydrogen atom or a straight-chain or branched (C₁-C₆)-alkyl radical that is unsubstituted or substituted by a phenyl radical,
X, Y, which are different, represent CO or SO₂
Z represents
- a straight-chain or branched (C₁-C₆)-alkyl radical substituted by one or two identical or different radicals (C₃-C₇)-cycloalkyl or (C₁-C₄)-trihaloalkyl,
- an adamant-1-yl radical,
- a phenyl radical that is unsubstituted or substituted by one or more identical or different halogen atoms or radicals straight-chain or branched (C₁-C₆)-alkyl, hydroxy, straight-chain or branched (C₁-C₆)-alkoxy, (C₁-C₄)-trihaloalkyl, cyano, 1,4-dihydropyridin-4-yl (unsubstituted or substituted by one or more identical or different radicals straight-chain or branched (C₁-C₆)-alkyl or straight-chain or branched (C₁-C₆)-alkoxycarbonyl), (3,5-di-tert-butyl-4-hydroxy)benzylcarbonylaminosulphonyl, (3,5-di-tert-butyl-4-hydroxy)benzoyloxy, (3-ethoxy-2-hydroxy)propoxy or [3,5-di-tert-butyl-4-(ethoxymethoxy)]benzyloxy,
- or a (C₁-C₄)-alkenyl radical substituted by a [3,5-di-tert-butyl-4-(ethoxymethoxy)]phenyl or (3,5-di-tert-butyl-4-hydroxy)phenyl radical,
c 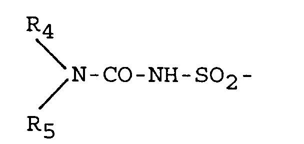 wherein :
R₅ represents a hydrogen atom,
R₄ represents phenyl (unsubstituted or substituted by one or more halogen atoms or radicals straight-chain or branched (C₁-C₆)-alkyl, straight-chain or branched (C₁-C₆)-alkoxy, C₁-C₄-trihaloalkyl),
or
form with the nitrogen atom to which they are attached the ring :
- 3-azabicyclo[3.3.0]octane,
f₁ 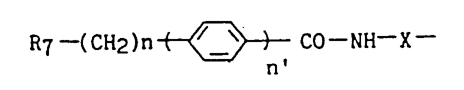
f₂ R₇-(CH₂)ₙ-
wherein :
X is as defined above,
n is 0, 1, 2 or 3,
n' is 0 or 1,
R₇ represents a radical :
. 3,5-di-tert-butyl-4-hydroxyphenyl,
. 3,5-di-tert-butyl-4-hydroxyphenoxy,
. or 3,5-di-tert-butyl-4-hydroxyphenylthio,
h 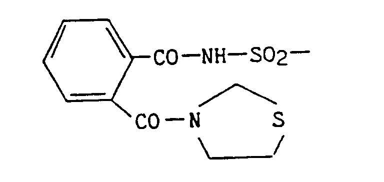
which compounds of formula (I) include the corresponding hydrates of the COCF₃ ketone function,
the enantiomers, diastereoisomers and epimers thereof, and also the addition salts thereof with a pharmaceutically acceptable acid or base.

2. Compounds of formula (I) according to claim 1 wherein B represents a radical 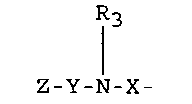 wherein :
R₃ represents a hydrogen atom or a straight-chain or branched (C₁-C₆)-alkyl radical that is unsubstituted or substituted by a phenyl radical,
X, Y , which are different, represent CO or SO₂,
Z represents
- a straight-chain or branched (C₁-C₆)-alkyl radical substituted by one or two identical or different radicals (C₃-C₇)-cycloalkyl or (C₁-C₄)-trihaloalkyl,
- an adamant-1-yl radical,
- a phenyl radical that is unsubstituted or substituted by one or more identical or different halogen atoms or radicals straight-chain or branched (C₁-C₆)-alkyl, hydroxy, straight-chain or branched (C₁-C₆)-alkoxy, (C₁-C₄)-trihaloalkyl, cyano, 1,4-dihydropyridin-4-yl (unsubstituted or substituted by one or more identical or different radicals straight-chain or branched (C₁-C₆)-alkyl or straight-chain or branched (C₁-C₆)-alkoxycarbonyl), (3,5-di-tert-butyl-4-hydroxy)benzylcarbonylaminosulphonyl, (3,5-di-tert-butyl-4-hydroxy)benzoyloxy, (3-ethoxy-2-hydroxy)propoxy or [3,5-di-tert-butyl-4-(ethoxymethoxy)]benzyloxy,
- or a (C₁-C₄)-alkenyl radical substituted by a [3,5-di-tert-butyl-4-(ethoxymethoxy)]phenyl or (3,5-di-tert-butyl-4-hydroxy)phenyl radical,
the hydrates, enantiomers, diastereoisomers and epimers thereof, and also the addition salts thereof with a pharmaceutically acceptable acid or base.

3. Compounds of formula (I) according to claim 1 wherein B represents a radical 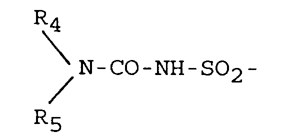 wherein :
R₅ represents a hydrogen atom,
R₄ represents phenyl (unsubstituted or substituted by one or more halogen atoms or radicals straight-chain or branched (C₁-C₆)-alkyl, straight-chain or branched (C₁-C₆)-alkoxy, (C₁-C₄)-trihaloalkyl),
or
form with the nitrogen atom to which they are attached the ring :
- 3-azabicyclo[3.3.0]octane,
the hydrates, enantiomers, diastereoisomers and epimers thereof, and also the addition salts thereof with a pharmaceutically acceptable acid or base.

4. Compounds of formula (I) according to claim 1, wherein B represents a radical 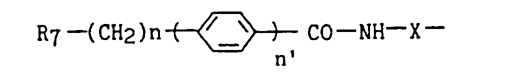 wherein :
X is as defined above,
n is 0, 1, 2 or 3,
n' is 0 or 1,
R₇ represents a radical :
. 3,5-di-tert-butyl-4-hydroxyphenyl,
. 3,5-di-tert-butyl-4-hydroxyphenoxy,
. or 3,5-di-tert-butyl-4-hydroxyphenylthio,
the hydrates, enantiomers, diastereoisomers and epimers thereof, and also the addition salts thereof with a pharmaceutically acceptable acid or base.

5. Compounds of formula (I) according to claim 1 wherein A represents, with the nitrogen and carbon atoms to which it is attached, a 2-azabicyclo[2.2.2]octane ring, the hydrates, enantiomers, diastereoisomers and epimers thereof, and also the addition salts thereof with a pharmaceutically acceptable acid or base.

6. Compounds of formula (I) according to claim 1 wherein A represents, with the nitrogen and carbon atoms to which it is attached, a perhydroindole ring, the hydrates, enantiomers, diastereoisomers and epimers thereof, and also the addition salts thereof with a pharmaceutically acceptable acid or base.

7. Compounds of formula (I) according to either claim 5 or claim 6 wherein B represents a 4-chlorobenzoylaminosulphonyl radical, the hydrates, enantiomers, diastereoisomers and epimers thereof, and also the addition salts thereof with a pharmaceutically acceptable acid or base.

8. Compounds of formula (I) according to either claim 5 or claim 6 wherein B represents a 4-chlorophenylsulphonylaminocarbonyl radical, the hydrates, enantiomers, diastereoisomers and epimers thereof, and also the addition salts thereof with a pharmaceutically acceptable acid or base.

9. Compound of formula (I) according to claim 1 that is 4-(4-chlorophenylsulphonylaminocarbonyl)benzoyl-(S)Val-(2S,3aS,7aS)Phi-(R,S)Val-CF₃, Phi representing a perhydroindole-2-carbonyl residue and Val a valyl residue, the hydrates, isomers and also the addition salts thereof with a pharmaceutically acceptable acid or base.

10. Compound of formula (I) according to claim 1 that is 4-(4-chlorophenylsulphonylaminocarbonyl)benzoyl-(S)Val-(S)Abo-(R,S)Val-CF₃, Abo representing a 2-azabicyclo[2.2.2]octane-3-carbonyl residue and Val a valyl residue, the hydrates, isomers and also the addition salts thereof with a pharmaceutically acceptable acid or base.

11. Process for the preparation of compounds of formula (I) according to claim 1, characterised in that there is used as starting material an alcohol of formula (II) : 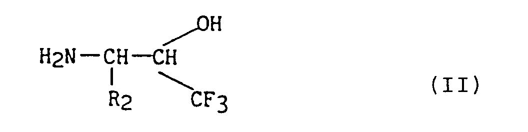 wherein R₂ is as defined for formula (I),
which has optionally been separated into its isomers by a conventional separation technique,
which is reacted :
a either with a protected amino acid of formula (III): 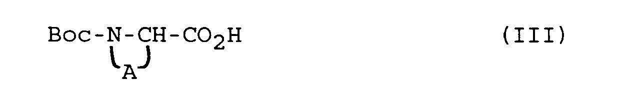 wherein A is as defined for formula (I) and Boc represents a butoxycarbonyl group,
which has optionally been separated into its isomers according to a conventional separation technique, by a conventional peptide coupling technique
to yield a compound of formula (IV) : 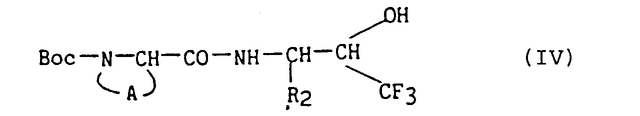 wherein A, R₂ and Boc are as defined above,
which compound of formula (IV) :
* either is deprotected by acid hydrolysis to yield a compound of formula (V) : 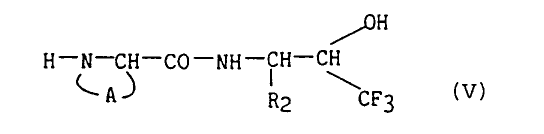 wherein A and R₂ are as defined above,
which is reacted in the presence of a conventional peptide synthesis coupling agent with a protected amino acid of formula (VI) : 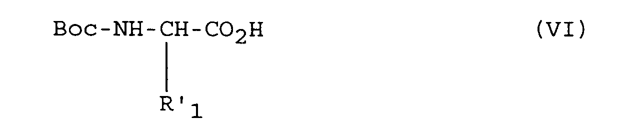 wherein Boc represents a butoxycarbonyl radical and R'₁ represents a straight-chain or branched (C₁-C₆)-alkyl radical that is unsubstituted or substituted by a (C₃-C₇)-cycloalkyl, phenyl or benzyloxycarbonylamino radical,
which has optionally been separated into its isomers according to a conventional separation technique,
to yield a compound of formula (VII): 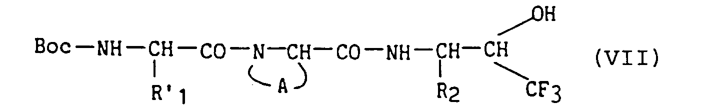 wherein Boc, R'₁, A and R₂ are as defined above,
which is subjected to an oxidation to yield a compound of formula (VIII) 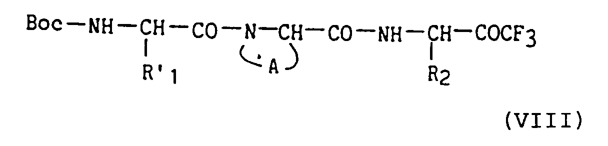 wherein Boc, R'₁, A and R₂ are as defined above, which is optionally separated into its isomers according to a conventional separation technique,
* or is subjected to an oxidation to yield a compound of formula (X) : 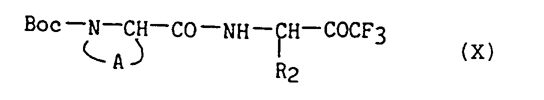 wherein Boc, A and R₂ are as defined above,
which is deprotected in acid medium to yield a compound of formula (XI) : 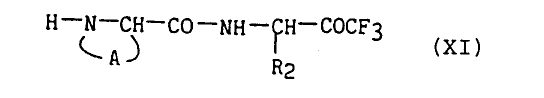 wherein A and R₂ are as defined above,
which is reacted with a protected amino acid of formula (VI) as defined above,
to yield a compound of formula (VIII) defined above,
b or with a protected dipeptide of formula (XII) : 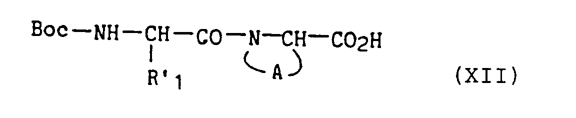 wherein Boc, R'₁ and A are as defined above,
obtained by conventional coupling of two amino acids in racemic form or in the form of pure enantiomers, to yield a compound of formula (VII) defined above, which is subjected to an oxidation and yields a compound of formula (VIII) defined above,
which compound of formula (VIII) is deprotected in acid medium,
to yield a compound of formula (IX), 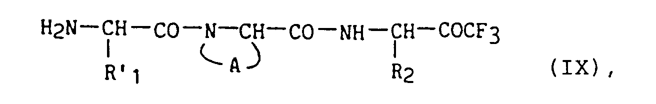 wherein R'₁, A and R₂ are as defined above, which is optionally separated into its isomers according to a conventional separation technique,
which is reacted according to a conventional peptide coupling technique with an acid of formula (XIII) 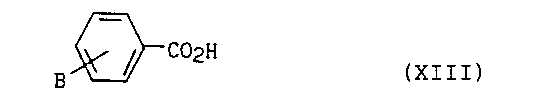 wherein B is as defined for formula (I),
to yield a compound of formula (I/a), a particular case of compounds of formula (I), 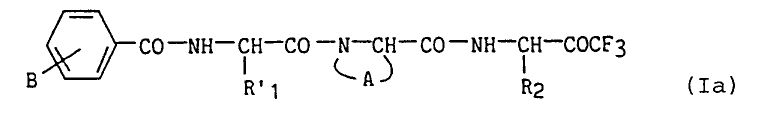 wherein B, A, R'₁ and R₂ are as defined above which, when R'₁ represents an alkyl radical substituted by a benzyloxycarbonylamino group, is deprotected, if desired, by catalytic hydrogenation,
to yield a compound of formula (I/b), a particular case of compounds of formula (I), 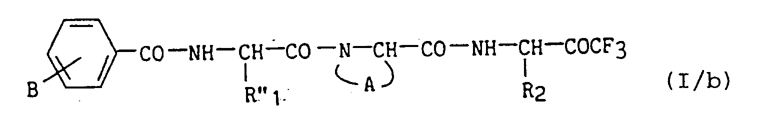 wherein B, A and R₂ are as defined for formula (I) and R''₁ represents an alkyl radical substituted by an amino radical,
which compounds of formula (I/a) and (I/b) are purified according to a conventional purification technique, are separated, if desired, into their isomers according to a conventional separation technique and then, if necessary, are converted into addition salts with a pharmaceutically acceptable acid or base.

12. Pharmaceutical compositions comprising as active ingredient at least one compound according to any one of claims 1 to 10, alone or in combination with one or more pharmaceutically acceptable, inert, non-toxic excipients or carriers.

13. Pharmaceutical compositions according to claim 12, comprising at least one active ingredient according to any one of claims 1 to 10 used as an inhibitor of human leucocyte elastase as a principal or complementary treatment for degenerative disorders of conjunctive tissue, such as pulmonary emphysema, inflammatory disorders, such as rheumatoid arthritis, pulmonary pathologies of the type acute respiratory distress syndrome, cystic fibrosis, chronic bronchitis, tissue lesions induced by ischaemia reperfusion syndrome and as a topical treatment on the skin for the purpose of inhibiting elastolysis.

## Patentansprüche

1. Verbindungen der Formel (I): 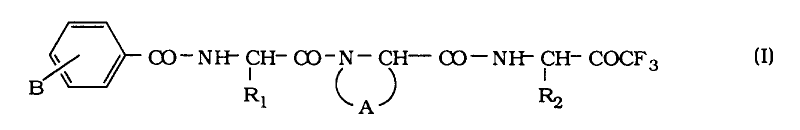 in der:
R₁ eine geradkettige oder verzweigte (C₁-C₆)-Alkylgruppe darstellt, die gegebenenfalls durch eine (C₃-C₇)-Cycloalkylgruppe, Phenylgruppe, Aminogruppe oder Benzyloxycarbonylaminogruppe, (3,5-Di-tert.-butyl-4-hydroxy)-benzylcarbonylaminogruppe, (3,5-Di-tert.-butyl-4-hydroxy)-phenylthiogruppe oder 3-[(3,5-Di-tert.-butyl-4-hydroxy)-phenylthio]-propylcarbonylaminogruppe substituiert ist,
R₂ eine geradkettige oder verzweigte (C₁-C₆)-Alkylgruppe darstellt,
A zusammen mit dem Stickstoff- und Kohlenstoffatom, an die es gebunden ist, einen 2-Azabicyclo[2.2.2]octanring, 2-Azabicyclo[2.2.1]heptanring oder Perhydroindolring bildet,
B eine der folgenden Gruppen darstellt:
a 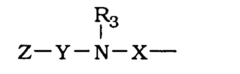 in der:
R₃ ein Wasserstoffatom oder eine geradkettige oder verzweigte, gegebenenfalls durch eine Phenylgruppe substituierte (C₁-C₆)-Alkylgruppe,
X, Y, die voneinander verschieden sind, CO oder SO₂ und
Z
- eine geradkettige oder verzweigte (C₁-C₆)-Alkylgruppe, die durch einen oder zwei gleichartige oder verschiedene (C₃-C₇)-Cycloalkyl- oder (C₁-C₄)-Trihalogenalkylgruppen substituiert ist,
- eine Adamant-1-yl-gruppe,
- eine Phenylgruppe, die gegebenenfalls durch eine oder mehrere gleichartige oder verschiedene Halogenatome oder geradkettige oder verzweigte (C₁-C₆)-Alkylgruppen, Hydroxylgruppen, geradkettige oder verzweigte (C₁-C₆)-Alkoxygruppen, (C₁-C₄)-Trihalogenalkylgruppen, Cyanogruppen, 1,4-Dihydropyridin-4-yl-gruppen (die gegebenenfalls durch eine oder mehrere gleichartige oder verschiedene geradkettige oder verzweigte (C₁-C₆)-Alkylgruppen oder geradkettige oder verzweigte (C₁-C₆)-Alkoxycarbonylgruppen substituiert ist), (3,5-Di-tert.-butyl-4-hydroxy)-benzylcarbonylaminosulfonyl-gruppen, (3,5-Di-tert.-butyl-4-hydroxy)-benzoyloxygruppen, (3-Ethoxy-2-hydroxy)-propoxy-gruppen oder [3,5-Di-tert.-butyl-4-(ethoxymethoxy)]-benzyloxygruppen substituiert ist, oder
- eine (C₁-C₄)-Alkenylgruppe, die durch eine [3,5-Di-tert.-butyl-4-(ethoxymethoxy)]-phenylgruppe oder (3,5-Di-tert.-butyl-4-hydroxyphenylgruppe substituiert ist, bedeuten,
c 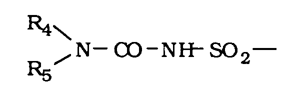 in der:
R₅ ein Wasserstoffatom,
R₄ eine Phenylgruppe (die gegebenenfalls durch eines oder mehrere Halogenatome, geradkettige oder verzweigte (C₁-C₆)-Alkylgruppen, geradkettige oder verzweigte (C₁-C₆)-Alkoxygruppen oder (C₁-C₄)-Trihalogenalkylgruppen substituiert ist), bedeuten,
oder
mit dem Stickstoffatom, an das sie gebunden sind, den
- 3-Azabicyclo[3.3.0]octanring bilden,
f₁ 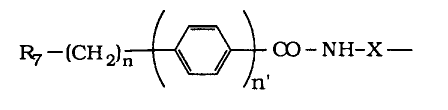
f₂ R₇―(CH₂)ₙ―
in denen:
X die oben angegebenen Bedeutungen besitzt,
n 0, 1, 2 oder 3,
n' 0 oder 1 und
R₇ eine:
. 3,5-Di-tert.-butyl-4-hydroxyphenylgruppe,
. 3,5-Di-tert.-butyl-4-hydroxyphenoxygruppe oder
. 3,5-Di-tert.-butyl-4-hydroxy-phenylthiogruppe
bedeuten,
h 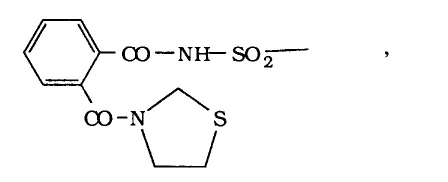
welche Verbindungen der Formel (I) die entsprechenden Hydrate der Ketonfunktion COCF₃ umfassen,
deren Enantiomere, Diastereoisomere und Epimere sowie deren Additionssalze mit einer pharmazeutisch annehmbaren Säure oder Base.

2. Verbindungen der Formel (I) nach Anspruch 1, worin B eine Gruppe 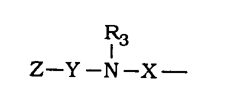 darstellt, in der:
R₃ ein Wasserstoffatom oder eine geradkettige oder verzweigte, gegebenenfalls durch eine Phenylgruppe substituierte (C₁-C₆)-Alkylgruppe,
X oder Y, die voneinander verschieden sind, CO oder SO₂ und
Z
- eine geradkettige oder verzweigte (C₁-C₆)-Alkylgruppe, die durch eine oder zwei gleichartige oder verschiedene (C₃-C₇)-Cycloalkylgruppen oder (C₁-C₄)-Trihalogenalkylgruppen substituiert ist,
- eine Adamant-1-yl-gruppe,
- eine Phenylgruppe, die gegebenenfalls durch eines oder mehrere, gleichartige oder verschiedene Halogenatome oder geradkettige oder verzweigte (C₁-C₆)-Alkylgruppen, Hydroxylgruppen, geradkettige oder verzweigte (C₁-C₆)-Alkoxygruppen, (C₁-C₄)-Trihalogenalkylgruppen, Cyanogruppen, 1,4-Dihydropyridin-4-yl-gruppen (die gegebenenfalls durch eine oder mehrere gleichartige oder verschiedene geradkettige oder verzweigte (C₁-C₆)-Alkylgruppen oder geradkettige oder verzweigte (C₁-C₆)-Alkoxycarbonylgruppen substituiert ist), (3,5-Di-tert.-butyl-4-hydroxy)-benzylcarbonylaminosulfonyl-gruppen, (3,5-Di-tert.-butyl-4-hydroxy)-benzoyloxy-gruppen, (3-Ethoxy-2-hydroxy)-propoxygruppen oder [3,5-Di-tert.-butyl-4-(ethoxymethoxy)]-benzyloxygruppen substituiert ist,
- oder eine (C₁-C₄)-Alkenylgruppen, die durch eine [3,5-Di-tert.-butyl-4-(ethoxymethoxy)]-phenylgruppe oder (3,5-Di-tert.-butyl-4-hydroxy)-phenylgruppe substituiert ist,
bedeuten,
deren Hydrate, Enantiomere, Diastereoisomere und Epimere sowie deren Additionssalze mit einer pharmazeutisch annehmbaren Säure oder Base.

3. Verbindungen der Formel (I) nach Anspruch 1, worin B eine Gruppe 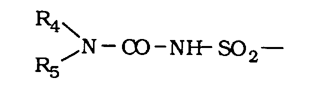 darstellt, in der:
R₅ ein Wasserstoffatom,
R₄ eine Phenylgruppe (die gegebenenfalls durch eines oder mehrere Halogenatome, geradkettige oder verzweigte (C₁-C₆)-Alkylgruppen, geradkettige oder verzweigte (C₁-C₆)-Alkoxygruppen oder (C₁-C₄)-Trihalogenalkylgruppen substituiert ist, bedeuten,
oder
gemeinsam mit dem Stickstoffatom, an das sie gebunden sind, den
- 3-Azabicyclo[3.3.0]octanring bilden,
deren Hydrate, Enantiomere, Diastereoisomere und Epimere sowie deren Additionssalze mit einer pharmazeutisch annehmbaren Säure oder Base.

4. Verbindungen der Formel (I) nach Anspruch 1, in denen B eine Gruppe 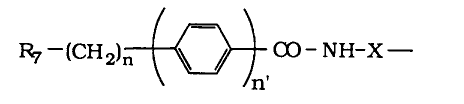 darstellt, in der:
X die oben angegebenen Bedeutungen besitzt und
n 0, 1, 2 oder 3,
n' 0 oder 1 und
R₇ eine:
. 3,5-Di-tert.-butyl-4-hydroxy-phenylgruppe,
. 3,5-Di-tert.-butyl-4-hydroxy-phenoxygruppe oder
. 3,5-Di-tert.-butyl-4-hydroxy-phenylthiogruppe
bedeuten,
deren Hydrate, Enantiomere, Diastereoisomere und Epimere sowie deren Additionssalze mit einer pharmazeutisch annehmbaren Säure oder Base.

5. Verbindungen der Formel (I) nach Anspruch 1, in denen A zusammen mit dem Stickstoff- und dem Kohlenstoffatom, an die es gebunden ist, einen 2-Azabicyclo[2.2.2]octanring bildet, deren Hydrate, Enantiomere, Diastereoisomere und Epimere sowie deren Additionssalze mit einer pharmazeutisch annehmbaren Säure oder Base.

6. Verbindungen der Formel (I) nach Anspruch 1, worin A zusammen mit dem Stickstoff- und dem Kohlenstoffatom, an die es gebunden ist, einen Perhydroindolring bildet, deren Hydrate, Enantiomere, Diastereoisomere und Epimere sowie deren Additionssalze mit einer pharmazeutisch annehmbaren Säure oder Base.

7. Verbindungen der Formel (I) nach irgendeinem der Ansprüche 5 oder 6, worin B eine 4-Chlorbenzoylaminosulfonylgruppe darstellt, deren Hydrate, Enantiomere, Diastereoisomere und Epimere sowie deren Additionssalze mit einer pharmazeutisch annehmbaren Säure oder Base.

8. Verbindungen der Formel (I) nach irgendeinem der Ansprüche 5 oder 6, worin B eine 4-Chlorpohenylsulfonylaminocarbonyl-gruppe darstellt, deren Hydrate, Enantiomere, Diastereoisomere und Epimere sowie deren Additionssalze mit einer pharmazeutisch annehmbaren Säure oder Base.

9. Verbindung der Formel (I) nach Anspruch 1, nämlich 4-(4-Chlorphenylsulfonylaminocarbonyl)-benzoyl-(S)Val-(2S,3aS,7aS)Phi-(R,S)Val-CF₃, worin Phi einen Perhydroindol-2-carbonylrest und Val einen Valylrest bedeuten, deren Hydrate und Ismere sowie deren Additionssalze mit einer pharmazeutisch annehmbaren Säure oder Base.

10. Verbindung der Formel (I) nach Anspruch 1, nämlich 4-(4-Chlorphenylsulfonylaminocarbonyl)-benzoyl-(S)Val-(S)Abo-(R,S)Val-CF₃, worin Abo einen 2-Azabicyclo[2.2.2]octan-3-carbonylrest und Val einen Valylrest bedeuten, deren Hydrate und Ismere sowie deren Additionssalze mit einer pharmazeutisch annehmbaren Säure oder Base.

11. Verfahren zur Herstellung der Derivate der Formel (I) nach Anspruch 1, **dadurch gekennzeichnet**, daß man als Ausgangsprodukt einen Alkohol der Formel (II), den man gegebenenfalls mit Hilfe einer klassischen Trennmethode in die Isomeren aufgetrennt hat, einsetzt: 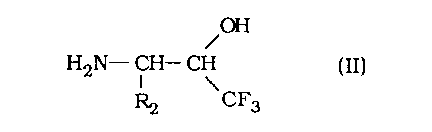 in der R₂ die bezüglich der Formel (I) angegebenen Bedeutungen besitzt, welchen man:
a entweder mit einer geschützten Aminosäure der Formel (III), die man gegebenenfalls mit Hilfe einer klassischen Trennmethode in die Isomeren aufgetrennt hat: 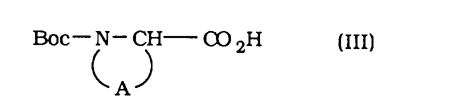 in der A die bezüglich der Formel (I) angegebenen Bedeutungen besitzt und Boc eine Butoxycarbonylgruppe darstellt, mit Hilfe einer klassischen Peptidkupplungstechnik umsetzt
zur Bildung der Verbindung der Formel (IV): 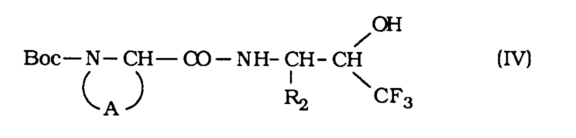 in der A, R₂ und Boc die oben angegebenen Bedeutungen besitzen,
welche Verbindung der Formel (IV):
* man entweder durch saure Hydrolyse von der Schutzgruppe befreit zur Bildung der Verbindung der Formel (V): 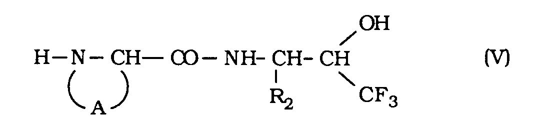 in der A und R₂ die oben angegebenen Bedeutungen besitzen,
welche man mit einer geschützten Aminosäure der Formel (VI), die man gegebenenfalls mit Hilfe einer klassischen Trennmethode in die Isomeren aufgetrennt hat: 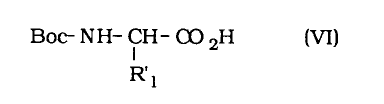 in der Boc eine Butoxycarbonylgruppe und R'₁ eine geradkettige oder verzweigte (C₁-C₆)-Alkylgruppe, die gegebenenfalls durch eine (C₃-C₇)-Cycloalkylgruppe, Phenylgruppe oder Benzyloxycarbonylaminogruppe substituiert ist, bedeuten,
in Gegenwart eines klassischen Kupplungsmittels für die Peptidsynthese umsetzt zur Bildung der Verbindung der Formel (VII): 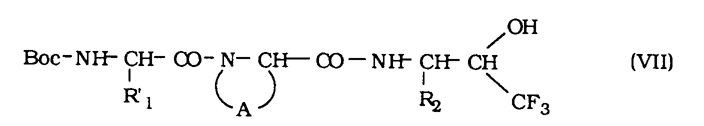 in der Boc, R'₁, A und R₂ die oben angegebenen Bedeutungen besitzen,
welche man einer Oxidation unterwirft zur Bildung der Verbindung der Formel (VIII): 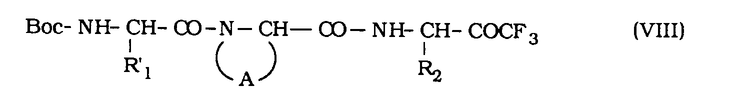 in der Boc, R'₁, A und R₂ die oben angegebenen Bedeutungen besitzen,
die man gegebenenfalls mit Hilfe einer klassischen Trennmethode in die Isomeren auftrennt,
* oder man einer Oxidation unterwirft zur Bildung der Verbindung der Formel (X): 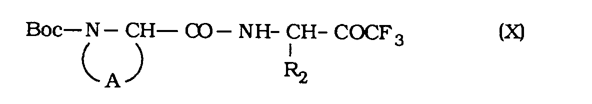 in der Boc, A und R₂ die oben angegebenen Bedeutungen besitzen,
von der man in saurem Medium die Schutzgruppe abspaltet zur Bildung der Verbindung der Formel (XI): 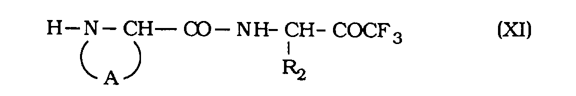 in der A und R₂ die oben angegebenen Bedeutungen besitzen,
welche man mit einer geschützten Aminosäure der oben definierten Formel (VI) umsetzt zur Bildung der Verbindung der oben definierten Formel (VIII),
b oder mit einem geschützten Dipeptid der Formel (XII), welches man durch klassische Kupplung von zwei Aminosäuren in Form des Racemats oder der reinen Enantiomeren erhalten hat, 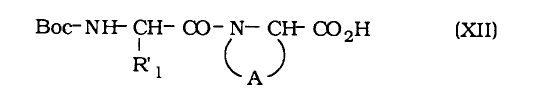 in der Boc, R'₁ und A die oben angegebenen Bedeutungen besitzen,
umsetzt zur Bildung der oben definierten Verbindung der Formel (VII), welche man einer Oxidation unterwirft zur Bildung der Verbindung der oben definierten Formel (VIII),
von welcher Verbindung der Formel (VIII) man die Schutzgruppe in saurem Medium abspaltet zur Bildung der Verbindung der Formel (IX), welche man gegebenenfalls mit Hilfe einer klassischen Trennmethode in die Isomeren auftrennt, 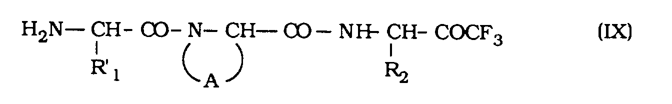 in der R'₁, A und R₂ die oben angegebenen Bedeutungen besitzen, welche man mit Hilfe einer klassischen Kupplungsmethode mit einer Säure der Formel (XIII) 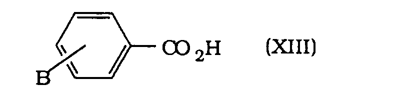 in der B die bezüglich der Formel (I) angegebenen Bedeutungen besitzt, umsetzt zur Bildung der Verbindung der Formel (I/a), einem Sonderfall der Verbindungen der Formel (I): 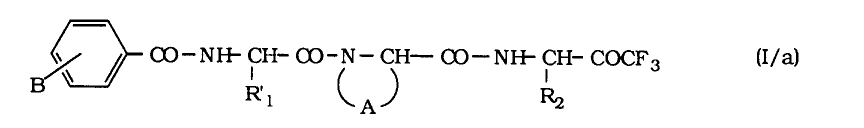 in der B, A, R'₁ und R₂ die oben angegebenen Bedeutungen besitzen, von welcher man, wenn R'₁ eine durch eine Benzyloxycarbonylaminogruppe substituierte Alkylgruppe darstellt, gewünschtenfalls die Schutzgruppe durch katalytische Hydrierung abspaltet,
zur Bildung der Verbindung der Formel (I/b), einem Sonderfall der Verbindungen der Formel (I): 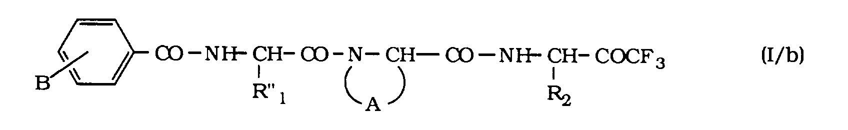 in der B, A und R₂ die bezüglich der Formel (I) angegebenen Bedeutungen besitzen und R''₁ eine durch eine Aminogruppe substituierte Alkylgruppe darstellt,
welche Verbindungen der Formel (I/a) und (I/b) man mit Hilfe einer klassischen Reinigungsmethode reinigt und gewünschtenfalls mit Hilfe einer klassischen Trennmethode in die Isomeren auftrennt und dann erforderlichenfalls in ein Additionssalze mit einer pharmazeutisch annehmbaren Säure oder Base umwandelt.

12. Pharmazeutische Zubereitungen enthaltend als Wirkstoff mindestens eine Verbindung nach irgendeinem der Ansprüche 1 bis 10 allein oder in Kombination mit einem oder mehreren inerten, nichttoxischen, pharmazeutisch annehmbaren Trägermaterialien oder Bindemitteln.

13. Pharmazeutische Zubereitungen nach Anspruch 12, enthaltend mindestens einen Wirkstoff gemäß irgendeinem der Ansprüche 1 bis 10 zur Verwendung als Inhibitor der humanen Leukozytenelastase bei der wesentlichen oder ergänzenden Behandlung von degenerativen Erkrankungen des Bindegewebes, wie Lungenemphysem, Entzündungszuständen, wie rheumatoider Arthritis, pathologischen Zuständen der Lunge des Typs des Syndroms der akuten Atemnot, der zystischen Fibrose, der chronischen Bronchitis, durch das Syndrom der Reperfusionsischämie verursachten Gewebeverletzungen sowie zur topischen Behandlung der Haut zur Inhibierung der Elastolyse.
